# EUROPEAN PATENT APPLICATION

(11) **EP 3 623 814 A2**
(43) Date of publication of application: **18.03.2020**
(21) Application number: 18798164.2
(22) Date of filing: 10.05.2018
(51) Int. Cl.: G01N 33/574

(54) **BIOMARKER FOR MONITORING OR DIAGNOSING ONSET OF LIVER CANCER IN HIGH-RISK GROUP FOR LIVER CANCER AND USE THEREOF**

(30) Priority: 10.05.2017 KR 20170058007
(71) Applicant: Seoul National University R&DB Foundation, Seoul 08826 (KR)
(72) Inventor: KIM, Youngsoo, Seoul 03080 (KR); LIM, Young-Suk, Seoul 05505 (KR); KIM, Hyunsoo, Seoul 03081 (KR); YEO, Injoon, Seoul 04808 (KR)
(74) Representative: Ter Meer Steinmeister & Partner
(86) International application number: PCT/KR2018/005353
(87) International publication number: WO 2018/208091

(57) **Abstract**

The present application discloses a biomarker capable of monitoring or diagnosing the onset of liver cancer at an early stage in a high-risk patient group for liver cancer and a method for monitoring, detecting, or diagnosing the onset of liver cancer at an early stage in high-risk group for liver cancer, using the marker. A biomarker according to the present application allows liver cancer to be accurately diagnosed through a simple blood test at an early stage in a high-risk patient group for liver cancer inclusive of liver cancer, hepatitis, and liver cirrhosis.

## Description

### BACKGROUND OF THE INVENTION

### Field of the Invention

The preset disclosure is related to monitoring or diagnosing onset of liver cancer in high-risk group for liver cancer.

### Description of the Related Art

Hepatocellular carcinoma (HCC) is a type ablof cancer which is most prevalent in adult liver cancer and is the 3^{rd} leading cause of cancer related death worldwide and accounts for over 90% of primary liver cancers(Ferrin, G.; Aguilar-Melero, P.; Rodriguez-Peralvarez, M.; Montero-Alvarez, J. L.; de la Mata, M. Hepatic medicine: evidence and research 2015, 7, 1-10).

Although studies on hepatocellular carcinoma continue and the symptoms are well known, it is still difficult to diagnose the disease at an early stage and the survival rate after diagnosis is very low (Xiao, J. F. et al., Journal ofproteome research 2012, 11, 5914-5923). For the complete removal of the cancer, surgery is required; however, the surgery is possible just in 10-20% of the cases.

Patients with cirrhosis or hepatitis B are clearly classified as a high-risk group for developing liver cancer. In hepatitis B, 75% of patients develop liver cancer through cirrhosis and 25% of patients directly develop liver cancer. The risk of developing liver cancer in patients with hepatitis B is 70-200 times higher than in the general population, and the annual incidence of liver cancer is 0.5-1%.

Therefore, in patients with hepatitis B or not so severe cirrhosis, the early diagnosis of liver cancer is very important in preventing liver cancer because the prognosis of patients has a great influence on the possibility of developing liver cancer.

Current diagnostic methods for liver cancer includes imaging methods such as ultrasonography, computed tomography and magnetic resonance imaging. However, the disadvantage is that the detection of small tumors is difficult and expensive. AFP is a main non-invasive method for early detection of liver cancer in people at high risk of developing the same. However, the specificity remained at just 50-60%. Further the level of AFP is increased in patients with benign liver diseases such as alcoholic hepatitis, viral hepatitis and cirrhosis, resulting in low specificity of 40-50%, which is insufficient for use as a biomarker for early diagnosis of liver cancer.

Especially when the disease progresses from cirrhosis to liver cancer, from hepatitis to liver cancer, i.e. from high risk to liver cancer, the liver to be diagnosed is at an very early stage, and thus it is difficult to detect or diagnose early liver cancer developed from hepatitis using the marker generally used to diagnose liver cancer at an advanced stage. For early liver cancer, the sensitivity of ultrasonography recommended for routine screening of the patients at high-risk of liver cancer is only 45%. Therefore, it is necessary to overcome the limitation of the individual test by a single marker by improving the accuracy of diagnosis by combining several markers with the continuous development of biomarkers with improved specificity and sensitivity suitable for detection of such liver cancer. Combining multiple biomarkers in distinct pathological physiological pathways has the potential to overcome these shortcomings and further enhance risk stratification, which can significantly improve risk prediction using multimarker approaches.

KR Patent Application Publication 10-2016-0072027 (Patent No: 10-1788414) discloses markers or combination of markers for diagnose, prognosis or monitoring liver cancer. However, the markers disclosed therein is difficult to use for monitoring or detecting patient at high risk of liver cancer or with early stage of liver cancer because the markers were developed in normal, liver cirrhosis and hepatitis B patients in comparison to the patients with advanced stage of liver cancer. KR Patent Application Publication 2015-0058465 discloses a method of assessing the risk of a subject with chronic hepatitis or liver cirrhosis to develop liver cancer.

### SUMMARY OF THE INVENTION

The present disclosure is to provide biomarkers which make possible the surveillance of the onset of liver cancer and early diagnosis of liver cancer in a high-risk group of patients developing liver cancer.

In one aspect, the present disclosure provides biomarkers for surveillance/monitoring the onset of liver cancer or early diagnosis of liver cancer in a high-risk group of patients developing liver cancer, and its use.

The present biomarkers may be employed as compositiona or kits comprising materials for detecting at least one of the present biomarkers in blood, or methods of surveillance/monitoring the onset of liver cancer or early diagnosis of liver cancer in a high-risk group of patients developing liver cancer, or methods of detecting at least one of the present biomarkers for surveillance/monitoring the onset of liver cancer or early diagnosis of liver cancer in a high-risk group of patients.

The present biomarkers are tested in a sample particularly from high-risk group of patients developing liver cancer, which includes hepatitis and liver cirrhosis patients.

The present biomarkers for surveillance/monitoring the onset of liver cancer or early diagnosis of liver cancer in a high-risk group of patients developing liver cancer are at least one markers selected from the group consisting of: ORM1 (Alpha-1-acid glycoprotein 1), SERPINA1 (Alpha-1-antitrypsin), SERPINF2 (Alpha-2-antiplasmin), A2M (Alpha-2-macroglobulin), ALB (Serum albumin), SERPINC1 (Antithrombin-III), APOA1 (Apolipoprotein A-I), APOA4 (Apolipoprotein A-IV), APOB (Apolipoprotein B-100), APOC3 (Apolipoprotein C-III), APOC4 (Apolipoprotein C-IV), APOF (Apolipoprotein F), APOH (Beta-2-glycoprotein 1), APOL1 (Apolipoprotein L1), APOM (Apolipoprotein M), BCO2 (Beta,beta-carotene 9',10'-oxygenase), BTD (Biotinidase), C1RL (Complement C1r subcomponent-like protein), CDH1 (Cadherin-1), CA1 (Carbonic anhydrase 1), CA2 (Carbonic anhydrase 2), CAT (Catalase), CPB2 (Carboxypeptidase B2), CETP (Cholesteryl ester transfer protein), CFH (Complement factor H), C3 (Complement C3), C8B (Complement component C8 beta chain), C9 (Complement component C9), PPBP (Platelet basic protein), HSP90B1 (Endoplasmin), ENTPD5 (Ectonucleoside triphosphate diphosphohydrolase 5), ESYT1 (Extended synaptotagmin-1), F7 (Coagulation factor VII), F9 (Coagulation factor IX), FABP1 (Fatty acid-binding protein, liver), FCGBP (IgGFc-binding protein), FETUB (Fetuin-B), FGA (Fibrinogen alpha chain), FGB (Fibrinogen beta chain), RACK1 (Receptor of activated protein C kinase 1), GP5 (Platelet glycoprotein V), GSS (Glutathione synthetase), SERPIND1 (Heparin cofactor 2), ICAM1 (Intercellular adhesion molecule 1), IGHM (Immunoglobulin heavy constant mu), IL1RAP (Interleukin-1 receptor accessory protein), ITIH2 (Inter-alpha-trypsin inhibitor heavy chain H2), ITIH3 (Inter-alpha-trypsin inhibitor heavy chain H3), KNG1 (Kininogen-1), LMNB1 (Lamin-B1), MPO (Myeloperoxidase), PON1 (Serum paraoxonase/arylesterase 1), PRDX2 (Peroxiredoxin-2), PROC (Vitamin K-dependent protein C), PROS1 (Vitamin K-dependent protein S), PROZ (Vitamin K-dependent protein Z), PSMD1 (26S proteasome non-ATPase regulatory subunit 1), PVR (Poliovirus receptor), RBP4 (Retinol-binding protein 4), S100A13 (Protein S100-A13), SAA4 (Serum amyloid A-4 protein), UBA2 (SUMO-activating enzyme subunit 2), SELENOP (Selenoprotein P), SPARC (SPARC), TF (Serotransferrin), THBS1 (Thrombospondin-1), UCHL3 (Ubiquitin carboxyl-terminal hydrolase isozyme L3), VCAM1 (Vascular cell adhesion protein 1), HDLBP (Vigilin), VIM (Vimentin), AZGP1 (Zinc-alpha-2-glycoprotein), SERPINA10 (Protein Z-dependent protease inhibitor), LRG1 (Leucine-rich alpha-2-glycoprotein), IGFALS (Insulin-like growth factor-binding protein complex acid labile subunit), AMBP (Protein AMBP [Cleaved into: Alpha-1-microglobulin), BCHE (Cholinesterase), CLU (Clusterin), CNDP1 (Beta-Ala-His dipeptidase), C6 (Complement component C6), C7 (Complement component C7), FBLN1 (Fibulin-1), FCGR3A (Low affinity immunoglobulin gamma Fc region receptor III-A), FCN3 (Ficolin-3), AFP (Alpha-fetoprotein), FN1 (Fibronectin), IGFBP3 (Insulin-like growth factor-binding protein 3), IGF2 (Insulin-like growth factor II), JCHAIN (Immunoglobulin J chain), SERPINA5 (Plasma serine protease inhibitor), ITIH1 (Inter-alpha-trypsin inhibitor heavy chain H1), SERPINA4 (Kallistatin), KLKB1 (Plasma kallikrein), LCAT (Phosphatidylcholine-sterol acyltransferase), LGALS3BP (Galectin-3-binding protein), LUM (Lumican), PLG (Plasminogen), QSOX1 (Sulfhydryl oxidase 1), SHBG (Sex hormone-binding globulin), F2 (Prothrombin), GC (Vitamin D-binding protein) and VTN (Vitronectin).

In one embodiment, the at least one markers are particularly selected from the group consisting of ORM1 (Alpha-1-acid glycoprotein 1), SERPINA1 (Alpha-1-antitrypsin), SERPINF2 (Alpha-2-antiplasmin), A2M (Alpha-2-macroglobulin), ALB (Serum albumin), SERPINC1 (Antithrombin-III), APOA1 (Apolipoprotein A-I), APOA4 (Apolipoprotein A-IV), APOB (Apolipoprotein B-100), APOC3 (Apolipoprotein C-III), APOC4 (Apolipoprotein C-IV), APOF (Apolipoprotein F), APOH (Beta-2-glycoprotein 1), APOL1 (Apolipoprotein L1), APOM (Apolipoprotein M), BCO2 (Beta,beta-carotene 9',10'-oxygenase), BTD (Biotinidase), C1RL (Complement C1r subcomponent-like protein), CDH1 (Cadherin-1), CA1 (Carbonic anhydrase 1), CA2 (Carbonic anhydrase 2), CAT (Catalase), CPB2 (Carboxypeptidase B2), CETP (Cholesteryl ester transfer protein), CFH (Complement factor H), C3 (Complement C3), C8B (Complement component C8 beta chain), C9 (Complement component C9), PPBP (Platelet basic protein), HSP90B1 (Endoplasmin), ENTPD5 (Ectonucleoside triphosphate diphosphohydrolase 5), ESYT1 (Extended synaptotagmin-1), F7 (Coagulation factor VII), F9 (Coagulation factor IX), FABP1 (Fatty acid-binding protein, liver), FCGBP (IgGFc-binding protein), FETUB (Fetuin-B), FGA (Fibrinogen alpha chain), FGB (Fibrinogen beta chain), RACK1 (Receptor of activated protein C kinase 1), GP5 (Platelet glycoprotein V), GSS (Glutathione synthetase), SERPIND1 (Heparin cofactor 2), ICAM1 (Intercellular adhesion molecule 1), IGHM (Immunoglobulin heavy constant mu), IL1RAP (Interleukin-1 receptor accessory protein), ITIH2 (Inter-alpha-trypsin inhibitor heavy chain H2), ITIH3 (Inter-alpha-trypsin inhibitor heavy chain H3), KNG1 (Kininogen-1), LMNB1 (Lamin-B1), MPO (Myeloperoxidase), PON1 (Serum paraoxonase/arylesterase 1), PRDX2 (Peroxiredoxin-2), PROC (Vitamin K-dependent protein C), PROS1 (Vitamin K-dependent protein S), PROZ (Vitamin K-dependent protein Z), PSMD1 (26S proteasome non-ATPase regulatory subunit 1), PVR PVS (Poliovirus receptor), RBP4 (Retinol-binding protein 4), S100A13 (Protein S100-A13), SAA4 (Serum amyloid A-4 protein), UBA2 (SUMO-activating enzyme subunit 2), SELENOP (Selenoprotein P), SPARC (SPARC), TF (Serotransferrin), THBS1 (Thrombospondin-1), UCHL3 (Ubiquitin carboxyl-terminal hydrolase isozyme L3), VCAM1 (Vascular cell adhesion protein 1), HDLBP (Vigilin), VIM (Vimentin), AZGP1 (Zinc-alpha-2-glycoprotein) and SERPINA10 (Protein Z-dependent protease inhibitor). In one embodiment, the markers may further comprise at least one markers selected from the group consisting of LRG1 (Leucine-rich alpha-2-glycoprotein), IGFALS (Insulin-like growth factor-binding protein complex acid labile subunit), AMBP (Protein AMBP [Cleaved into: Alpha-1-microglobulin), BCHE (Cholinesterase), CLU (Clusterin), CNDP1 (Beta-Ala-His dipeptidase), C6 (Complement component C6), C7 (Complement component C7), FBLN1 (Fibulin-1), FCGR3A (Low affinity immunoglobulin gamma Fc region receptor III-A), FCN3 (Ficolin-3), AFP (Alpha-fetoprotein), FN1 (Fibronectin), IGFBP3 (Insulin-like growth factor-binding protein 3), IGF2 (Insulin-like growth factor II), JCHAIN (Immunoglobulin J chain), SERPINA5 (Plasma serine protease inhibitor), ITIH1 (Inter-alpha-trypsin inhibitor heavy chain H1), SERPINA4 (Kallistatin), KLKB1 (Plasma kallikrein), LCAT (Phosphatidylcholine-sterol acyltransferase), LGALS3BP (Galectin-3-binding protein), LUM (Lumican), PLG (Plasminogen), QSOX1 (Sulfhydryl oxidase 1), SHBG (Sex hormone-binding globulin), F2 (Prothrombin), GC (Vitamin D-binding protein) and VTN (Vitronectin).

In one embodiment, the markers for surveillance/monitoring the onset of liver cancer or early diagnosis of liver cancer in a high-risk group of patients developing liver cancer are at least one markers selected from the group consisting of SERPINA1, ALB, APOB, APOC3, APOC4, APOL1, BTD, CDH1, CA1, CAT, CETP, C3, ESYT1, FCGBP, FETUB, FGA, GP5, GSS, SERPIND1, IL1RAP, ITIH2, LMNB1 MPO, PON1, PRDX2, PVR, SAA4, UBA2, SELENOP, TF, THBS1, UCHL3, HDLBP, AZGP1 and SERPINA10.

In one embodiment, the markers for surveillance/monitoring the onset of liver cancer or early diagnosis of liver cancer in a high-risk group of patients developing liver cancer may further comprise at least one marker selected from the group consisting of LRG1, C7, AFP, JCHAIN, SERPINA5, SERPINA4, LGALS3BP, LUM, QSOX1, SHBG, F2, and VTN.

In one embodiment, the high-risk group of patients developing liver cancer is a hepatitis patients, and the markers which may be used for surveillance/monitoing the onset of liver cancer or early diagnosis of liver cancer for hepatitis patients are at least one markers selected from the group consisting of ORM1, A2M, ALB, SERPINC1, APOA1, APOA4, APOB, APOC3, APOC4, APOF, APOL1, APOH, APOM, BCO2, CDH1, CPB2, CA1, CA2, CAT, CETP, C8B, PPBP, F9, FABP1, ENTPD5, F7, FCGBP, FETUB, FGA, RACK1, GP5, SERPIND1, ICAM1, IGF2, IL1RAP, ITIH2, ITIH3, KNG1, PON1, PROC, PROS1, PROZ, PSMD1, RBP4, S100A13, SAA4, SELENOP, SPARC, THBS1, VCAM1, VIM and SERPINA10.

In one embodiment, the markers for surveillance/monitoring the onset of liver cancer or early diagnosis of liver cancer in hepatitis patients may further comprise at least one markers selected from the group consisting of IGFALS, BCHE, CLU, CNDP1, C7, FCGR3A, FCN3, AFP, IGFBP3, ITIH1, SERPINA4, KLKB1, LCAT, LGALS3BP, PLG, QSOX1, SHBG, F2, GC and VTN.

In one embodiment, the high-risk group of patients developing liver cancer is a liver cirrhosis patients, and the markers which may be used for surveillance/monitoring the onset of liver cancer or early diagnosis of liver cancer for liver cirrhosis patients are at least one markers selected from the group consisting of SERPINF2, APOA4, APOB, APOL1, APOM, BCO2,C1RL, CA2, CAT, CETP, CFH, C9, PPBP, HSP90B1, F9, FABP1, FGA, FGB, GSS, SERPIND1, IGHM, ITIH2, MPO, PON1, RBP4, SELENOP, UCHL3, HDLBP, AZGP1 and SERPINA10.

In one embodiment, the markers for surveillance/monitoring the onset of liver cancer or early diagnosis of liver cancer in liver cirrhosis patients may further comprise at least one markers selected from the group consisting of AMBP, BCHE, CLU, C6, C7, FBLN1, AFP, FN1, SERPINA5, SERPINA4, LUM, QSOX1, SHBG, and F2.

In one embodiment, the present markers are used in combination of two or markers for surveillance/monitoring the onset of liver cancer or early diagnosis of liver cancer in high-risk group of developing liver cancer, the combinations are ALB, APOB, APOL1, BTD, CDH1, CAT, CETP, ESYT1, AFP, FGA, GP5, GSS, SERPINA5, ITIH2, ITIH2, ERPINA4, LUM, MPO, PON1, PVR, SAA4, and SERPINA10; APOB, CAT, CETP, C7, AFP, FETUB, FGA, GP5, SERPIND1, SERPINA5, ITIH2, SERPINA4, LGALS3BP, MPO, QSOX1, SHBG, TF, VTN, AZGP1, and SERPINA10; LRG1, APOC3, CETP, AFP, SERPINA5, TF, and SERPINA10; APOC4, AFP, SERPINA5, LMNB1, MPO, PVR, and SERPINA10; SERPINA1, APOB, CAT, CETP, AFP, FETUB, FGA, GSS, SERPINA5, ITIH2, SERPINA4, UBA2, SHBG, F2, THBS1, THBS1, and SERPINA10; CETP, C7, AFP, IL1RAP, SERPINA5, LGALS3BP, MPO, HDLBP, and SERPINA10; ALB, APOB, CA1, CETP, C3, FCGBP, AFP, FETUB, FGA, JCHAIN, SERPINA5, ITIH2, SERPINA4, PRDX2, SELENOP, SHBG, THBS1, UCHL3, and SERPINA10; or ALB, APOB, CA1, CETP, AFP, FETUB, SERPINA5, ITIH2, SERPINA4, MPO, F2, THBS1, and SERPINA10, in particular, the combinations of two or more markers are APOB, CAT, CETP, C7, AFP, FETUB, FGA, GP5, SERPIND1, SERPINA5, ITIH2, SERPINA4, LGALS3BP, MPO, QSOX1, SHBG, TF, VTN, AZGP1 and SERPINA10; or ALB, APOB, CA1, CETP, C3, FCGBP, AFP, FETUB, FGA, JCHAIN, SERPINA5, ITIH2, SERPINA4, PRDX2, SELENOP, SHBG, THBS1, UCHL3, and SERPINA10.

In one embodiment, the present markers are used in combination of two or more markers for surveillance/monitoring the onset of liver cancer or early diagnosis of liver cancer particularly in hepatitis patients belong to high-risk group of developing liver cancer, the combinations are IGFALS, APOA4, C7, PPBP, F9, FABP1, FCGBP, AFP, ITIH1, SERPINA4, and SERPINA10; IGFALS, APOB, APOC3, PPBP, FABP1, FCGBP, AFP, ITIH1, SERPINA4, PROS1, and SERPINA10; ALB, IGFALS, CETP, C7, PPBP, FABP1, AFP, IL1RAP, ITIH1, ITIH2, SERPINA4, S100A13, and SERPINA10; ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10; IGFALS, CA2, CETP, C7, PPBP, FABP1, FCGBP, FCN3, AFP, FGA, ITIH1, SERPINA4, and SERPINA10; IGFALS, SERPINC1, APOA1, CAT, C7, PPBP, F9, FCGBP, AFP, FGA, ITIH2, SERPINA4, PROZ, QSOX1, and SERPINA10; APOB, APOC4, APOH, CDH1, PPBP, FABP1, AFP, FETUB, SERPINA4, and SERPINA10; or APOB, APOC4, APOH, APOM, CAT, PPBP, FABP1, AFP, FETUB, ITIH1, SERPINA4, and SERPINA10, in particular, the combinations of two or more markers are IGFALS, APOB, APOC3, PPBP, FABP1, FCGBP, AFP, ITIH1, SERPINA4, PROS1, and SERPINA10; or ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10.

In one embodiment, the present markers are used in combination of two or more markers for surveillance/monitoring the onset of liver cancer or early diagnosis of liver cancer particularly in liver cirrhosis patients belong to high-risk group of developing liver cancer, the combinations are APOB, APOL1, CAT, CETP, C7, FABP1, AFP, FGA, FN1, IGHM, SERPINA5, ITIH2, SERPINA4, PON1, PON1, and SERPINA10; BCO2,CAT, CETP, CFH, BCHE, AFP, FGA, SERPINA5, SELENOP, UCHL3, AZGP1, and SERPINA10; AMBP, APOB, APOM, C1RL, CAT, CETP, CLU, PPBP, HSP90B1, F9, FGA, GSS, SERPINA5, SERPINA4, RBP4, HDLBP, AZGP1, and SERPINA10; APOA4, APOB, C1RL, CETP, AFP, FGB, SERPIND1, SERPINA5, SERPINA4, LUM, QSOX1, SHBG, and SERPINA10; APOA4, APOB, APOL1, CETP, C9, FBLN1, AFP, FGA, SERPINA5, ITIH2, and SERPINA10; SERPINF2, APOB, CETP, FBLN1, AFP, FGA, SERPINA5, RBP4, HDLBP, and SERPINA10; APOB, CETP, AFP, FGA, SERPINA5, SERPINA4, F2, and SERPINA10; or APOA4, APOB, APOL1, CA2, CETP, CLU, C6, AFP, FGA, SERPINA5, ITIH2, SERPINA4, AZGP1, and SERPINA10, in particular, the combinations of two or more markers are BCO2, CAT, CETP, CFH, BCHE, AFP, FGA, SERPINA5, SELENOP, UCHL3, AZGP1, and SERPINA10; or APOA4, APOB, C1RL, CETP, AFP, FGB, SERPIND1, SERPINA5, SERPINA4, LUM, QSOX1, SHBG, and SERPINA10.

The present markers are detected in the blood samples including such as plasma, serum or whole blood. In one embodiment, the present markers are tested particularly in plasma sample.

The present biomarkers may be provided as compositions or kits for ELISA dip stick rapid kit, mass spectrometry, microarray, nucleic amplification or immunoassays.

In one embodiment, the present biomarkers are provided as compositions or kits for mass spectrometry, particularly MRM analysis. The peptides from each of the present markers used for MRM analysis are the ones listed in Table 1-1, 1-2 and 1-3.

In other aspect, the present disclosure provides a method of surveillance/monitoring the onset of liver cancer or early diagnosis of liver cancer in a high-risk group of patients developing liver cancer comprising the step of detecting the markers disclosed herein or the combinations thereof, or method of detecting the present biomarkers or the combination of the markers disclosed herein for surveillance/monitoring the onset of liver cancer or early diagnosis of liver cancer in a high-risk group of patients.

In one embodiment, the methods are for surveillance/monitoring the onset of liver cancer or early diagnosis of liver cancer in a high-risk group of patients developing liver cancer, the method comprising the steps of detecting or quantifying the present markers or combinations thereof in a sample from a subject in need thereof and comparing the result from the previous step to that from the corresponding markers in control sample; and determining that the sample or the subject is afflicted with early liver cancer when there are changes in the result from the subject in comparison to that from control, wherein the control sample is a sample from a hepatitis B patients or cirrhosis patients without liver cancer or having not developed liver cancer.

The present biomarkers may be detected by ELISA, dip stick rapid kit, mass spectrometry, microarray, nucleic amplification or immunoassays and the like.

In one embodiment, the present biomarkers are analyzed in samples using particularly mass spectrometry, more particularly MRM. The peptides for each marker which may be used in MRM analysis are listed in Tables 1-1, 1-2 and 1-3.

### ADVANTAGEOUS EFFECTS

The present biomarkers and the methods of using the same can accurately diagnose the risk of developing liver cancer or liver cancer at early stage in high-risk group of patients (hepatitis B, cirrhosis). In particular, the current clinical guideline for early diagnosis of hepatocellular carcinoma in high-risk group of patients with hepatitis B and cirrhosis is to visit a clinic every six months for ultrasound and AFP test. However, ultrasound examinations require a specialist and the degree of diagnosis may vary widely depending on the proficiency level. Therefore, by employing the marker according to the present disclosure, it is possible for surveillance/monitoring and early diagnosis of the onset of liver cancer at early stage including extremely early stage by detecting the expression level of the markers or combination of the markers in patients with high risk of developing liver cancer including hepatitis and cirrhosis patients.

Also, when MRM (Multiple Reaction Monitoring) based on mass spectrometry is used, the peptides for each markers of the present disclosure are easy to be applied as MRM assays which can replace the conventional immunoassays currently used in clinical fields. And thus, the accuracy of the diagnosis of liver cancer, especially liver cancer at early stage, can be significantly improved.

In addition, the marker according to the present disclosure is very useful for early detection of liver cancer for home and general clinical use in a non-invasive way using blood. The patient's pain and economic burden can be reduced by testing liver cancer through a simple blood test during the medical examination, which can bring about a medical cost reduction from a national perspective.

### BRIEF DESCRIPTION OF THE DRAWINGS

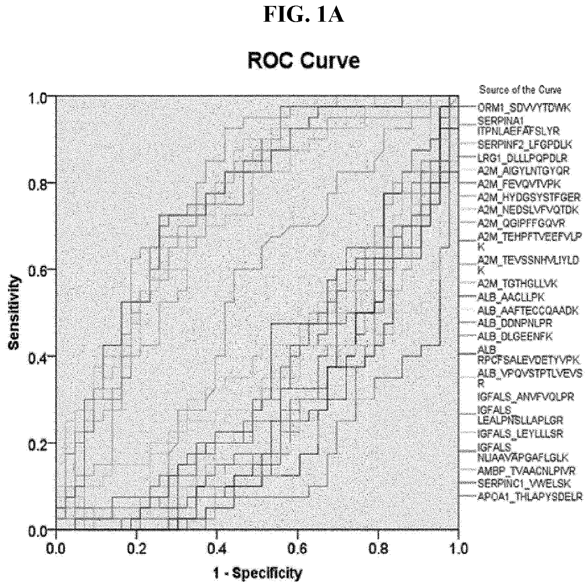

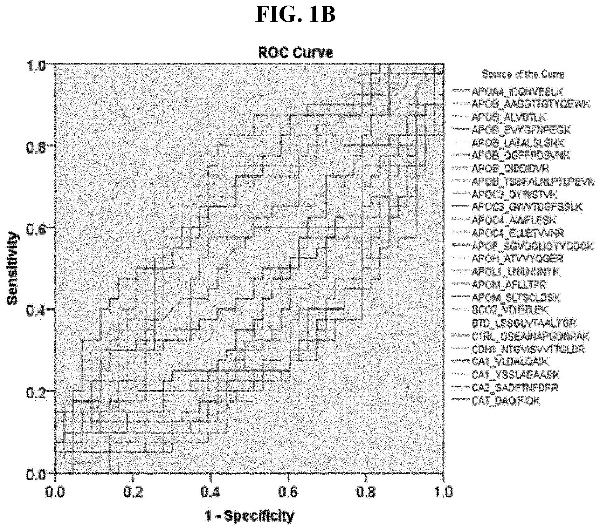

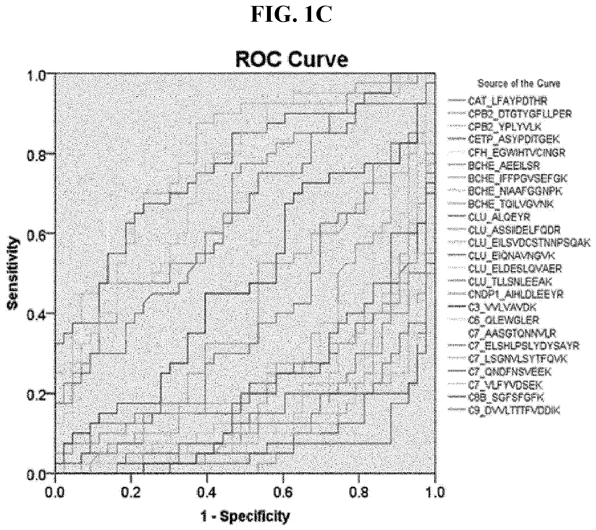

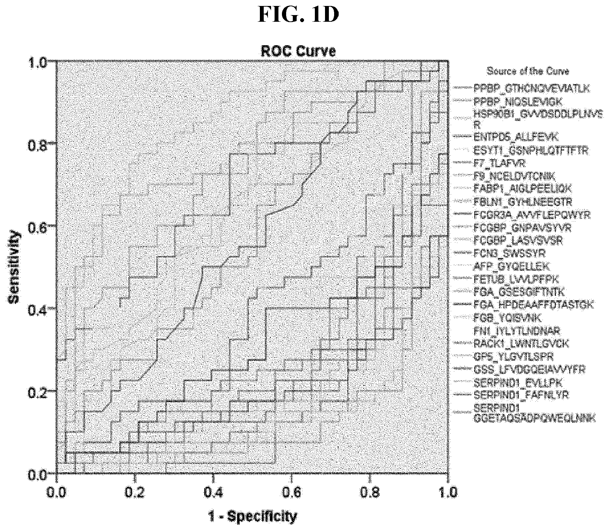

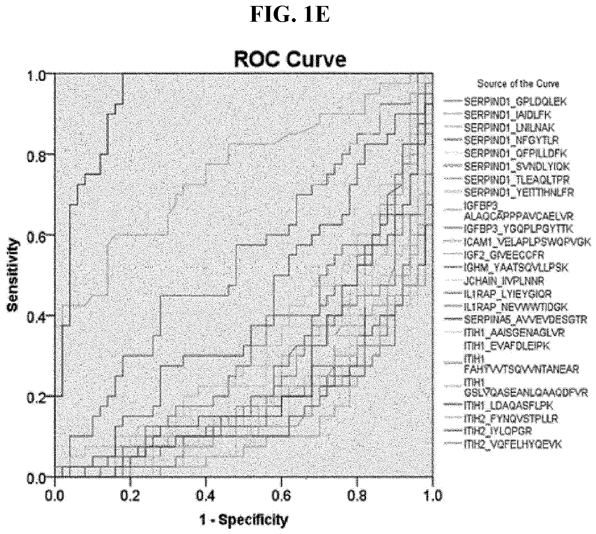

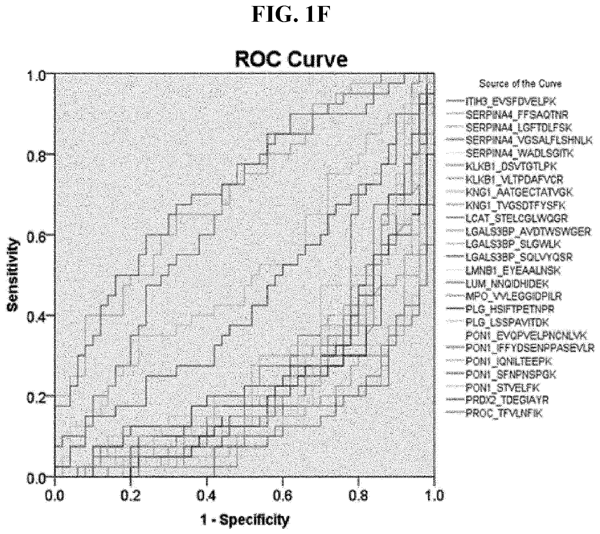

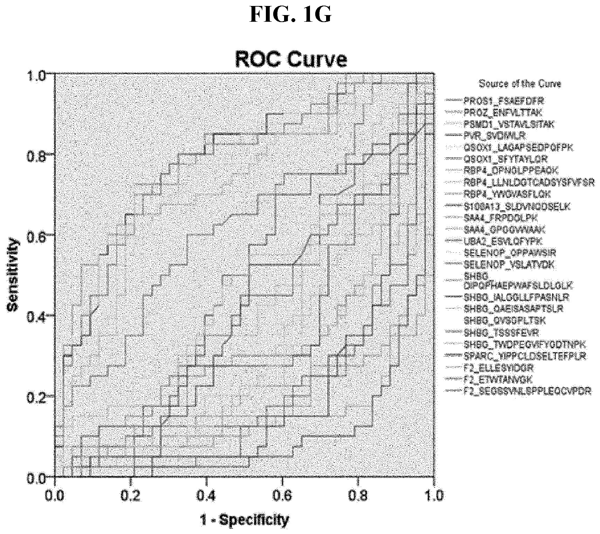

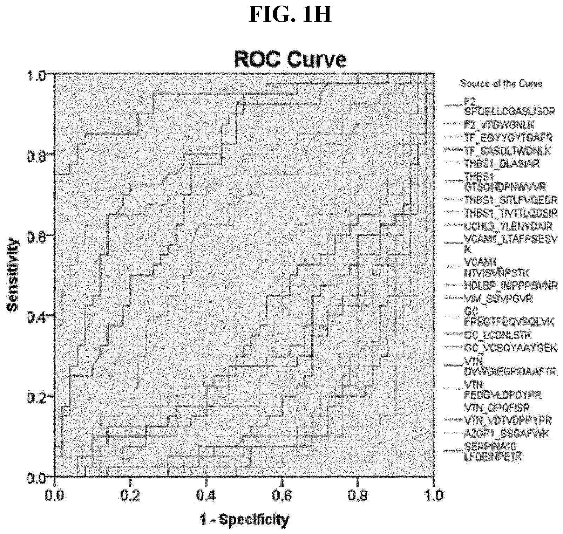

FIG. 1a, FIG.1b, FIG.1c, FIG.1d, FIG.1e, FIG. If, FIG.1g and FIG.1h are the ROC (Receiver Operating Characteristic) analysis results of the present biomarkers (refer to Table 1-1, 1-2 and 1-3) that showed the expression level different from that of the control. For ROC analysis, refer to Zweig MH, Campbell G (1993) Receiver-operating characteristic (ROC) plots: a fundamental evaluation tool in clinical medicine. Clinical Chemistry 39:561-577. ROC analysis is generally used to make decisions about the accuracy or effectiveness of a particular marker for the diagnosis and prognosis of disease. In ROC curve analysis, the model is evaluated based on sensitivity and specificity, and the area under curve (AUC) calculated from the ROC curve representing degree or measure of the sensitivity and specificity is used to confirm the diagnostic accuracy of a particular marker. The results show the high degree of diagnostic accuracy of the present biomarkers. In the figures, diagonal segments were produced by ties.

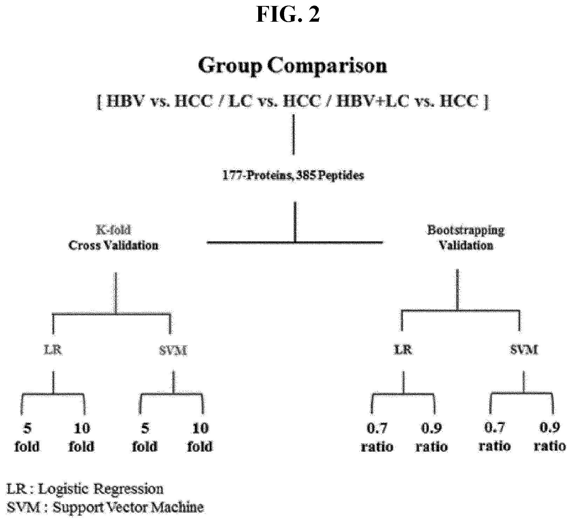

FIG. 2 schematically shows a multivariate analysis strategy in accordance with one embodiment of the present disclosure. In the present disclosure, logistic regression and support vector machine method were used for the analysis. In order to prevent overfitting, resampling was used to secure the statistical reliability of the analysis results. In the resampling method, 5- and 10-fold cross validation) (Kim H. et al., Sci Rep. 2017 Aug 25;7(1):9449 and Kim H. et al., Mol Cell Proteomics. 2017 Jul;16(7): 1312-1323) were used. In the bootstrap method, data used were divided at a ratio of 0.7 and 0.9.

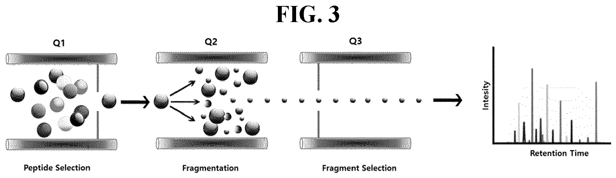

FIG.3 is a schematic diagram of MRM analysis.

### DETAILED DESCRIPTION OF THE EMBODIMENTS

The present disclosure is based in part on the discovery of the markers that show differential expression in blood sample between Hepatitis B and Liver cirrhosis group classified as a high-risk group of developing liver cancer and liver cancer group at a very early stage (less than 5cm in diameter) and thus can be advantageously used for early detection or diagnosis of liver cancer in high-risk group.

Therefore, in one aspect of the present disclosure, there is provided at least one markers or the composition comprising the material for detecting the at least one markers in blood sample for early diagnosis of liver cancer of the subject at high risk of developing liver cancer.

The at least one marker in the present disclosure is selected from the group consisting of ORM1 (Alpha-1-acid glycoprotein 1), SERPINA1 (Alpha-1-antitrypsin), SERPINF2 (Alpha-2-antiplasmin), A2M (Alpha-2-macroglobulin), ALB (Serum albumin), SERPINC1 (Antithrombin-III), APOA1 (Apolipoprotein A-I), APOA4 (Apolipoprotein A-IV), APOB (Apolipoprotein B-100), APOC3 (Apolipoprotein C-III), APOC4 (Apolipoprotein C-IV), APOF (Apolipoprotein F), APOH (Beta-2-glycoprotein 1), APOL1 (Apolipoprotein L1), APOM (Apolipoprotein M), BCO2 (Beta,beta-carotene 9',10'-oxygenase), BTD (Biotinidase), C1RL (Complement C1r subcomponent-like protein), CDH1 (Cadherin-1), CA1 (Carbonic anhydrase 1), CA2 (Carbonic anhydrase 2), CAT (Catalase), CPB2 (Carboxypeptidase B2), CETP (Cholesteryl ester transfer protein), CFH (Complement factor H), C3 (Complement C3), C8B (Complement component C8 beta chain), C9 (Complement component C9), PPBP (Platelet basic protein), HSP90B1 (Endoplasmin), ENTPD5 (Ectonucleoside triphosphate diphosphohydrolase 5), ESYT1 (Extended synaptotagmin-1), F7 (Coagulation factor VII), F9 (Coagulation factor IX), FABP1 (Fatty acid-binding protein, liver), FCGBP (IgGFc-binding protein), FETUB (Fetuin-B), FGA (Fibrinogen alpha chain), FGB (Fibrinogen beta chain), RACK1 (Receptor of activated protein C kinase 1), GP5 (Platelet glycoprotein V), GSS (Glutathione synthetase), SERPIND1 (Heparin cofactor 2), ICAM1 (Intercellular adhesion molecule 1), IGHM (Immunoglobulin heavy constant mu), IL1RAP (Interleukin-1 receptor accessory protein), ITIH2 (Inter-alpha-trypsin inhibitor heavy chain H2), ITIH3 (Inter-alpha-trypsin inhibitor heavy chain H3), KNG1 (Kininogen-1), LMNB1 (Lamin-Bl), MPO (Myeloperoxidase), PON1 (Serum paraoxonase/arylesterase 1), PRDX2 (Peroxiredoxin-2), PROC (Vitamin K-dependent protein C), PROS1 (Vitamin K-dependent protein S), PROZ (Vitamin K-dependent protein Z), PSMD1 (26S proteasome non-ATPase regulatory subunit 1), PVR (Poliovirus receptor), RBP4 (Retinol-binding protein 4), S100A13 (Protein S100-A13), SAA4 (Serum amyloid A-4 protein), UBA2 (SUMO-activating enzyme subunit 2), SELENOP (Selenoprotein P), SPARC (SPARC), TF (Serotransferrin), THBS1 (Thrombospondin-1), UCHL3 (Ubiquitin carboxyl-terminal hydrolase isozyme L3), VCAM1 (Vascular cell adhesion protein 1), HDLBP (Vigilin), VIM (Vimentin), AZGP1 (Zinc-alpha-2-glycoprotein) and SERPINA10 (Protein Z-dependent protease inhibitor), LRG1 (Leucine-rich alpha-2-glycoprotein), IGFALS (Insulin-like growth factor-binding protein complex acid labile subunit), AMBP (Protein AMBP [Cleaved into: Alpha-1-microglobulin), BCHE (Cholinesterase), CLU (Clusterin), CNDP1 (Beta-Ala-His dipeptidase), C6 (Complement component C6), C7 (Complement component C7), FBLN1 (Fibulin-1), FCGR3A (Low affinity immunoglobulin gamma Fc region receptor III-A), FCN3 (Ficolin-3), AFP (Alpha-fetoprotein), FN1 (Fibronectin), IGFBP3 (Insulin-like growth factor-binding protein 3), IGF2 (Insulin-like growth factor II), JCHAIN (Immunoglobulin J chain), SERPINA5 (Plasma serine protease inhibitor), ITIH1 (Inter-alpha-trypsin inhibitor heavy chain H1), SERPINA4 (Kallistatin), KLKB1 (Plasma kallikrein), LCAT (Phosphatidylcholine-sterol acyltransferase), LGALS3BP (Galectin-3-binding protein), LUM (Lumican), PLG (Plasminogen), QSOX1 (Sulfhydryl oxidase 1), SHBG (Sex hormone-binding globulin), F2 (Prothrombin), GC (Vitamin D-binding protein) and VTN (Vitronectin).

In one embodiment of the present disclosure, the at least one markers is selected from the group consisting of ORM1, SERPINA1, SERPINF2, A2M, ALB, SERPINC1, APOA1, APOA4, APOB, APOC3, APOC4, APOF, APOH, APOL1, APOM, BCO2, BTD, C1RL, CDH1, CA1, CA2, CAT, CPB2, CETP, CFH, C3 (Complement C3), C8B, C9, PPBP, HSP90B1, ENTPD5, ESYT1, F7, F9, FABP1, FCGBP, FETUB, FGA, FGB, RACK1, GP5, GSS, SERPIND1, ICAM1, IGHM, IL1RAP, ITIH2, ITIH3, KNG1, LMNB1, MPO, PON1, PRDX2, PROC, PROS1, PROZ, PSMD1, PVR, RBP4, S100A13, SAA4, UBA2, SELENOP, SPARC, TF, THBS1, UCHL3, VCAM1, HDLBP, VIM, AZGP1 and SERPINA10. In other embodiment of the present disclosure, the at least one markers is selected from the group consisting of LRG1, IGFALS, AMBP, BCHE, CLU, CNDP1, C6, C7, FBLN1, FCGR3A, FCN3, AFP, FN1, IGFBP3, IGF2, JCHAIN, SERPINA5, ITIH1, SERPINA4, KLKB1, LCAT, LGALS3BP, LUM, PLG, QSOX1, SHBG, F2, GC and VTN.

The biomarkers or markers of the present disclosure are markers for early detection of liver cancer in patients with high-risk for developing liver cancer, and include proteins, polypeptides derived from the proteins, gene encoding the protein or fragments thereof which show a change in concentrations in samples, especially blood, derived from patients with liver cancer compared to control samples.

The biomarker according to the present disclosure has an increased or decreased amount in the blood of liver cancer patients compared to the control sample, and the sequences for the markers can be found/searched for example in the UniProt (www.uniprot.org) with IDs disclosed in Tables 1-1, 1-2 and 1-3.

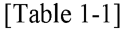

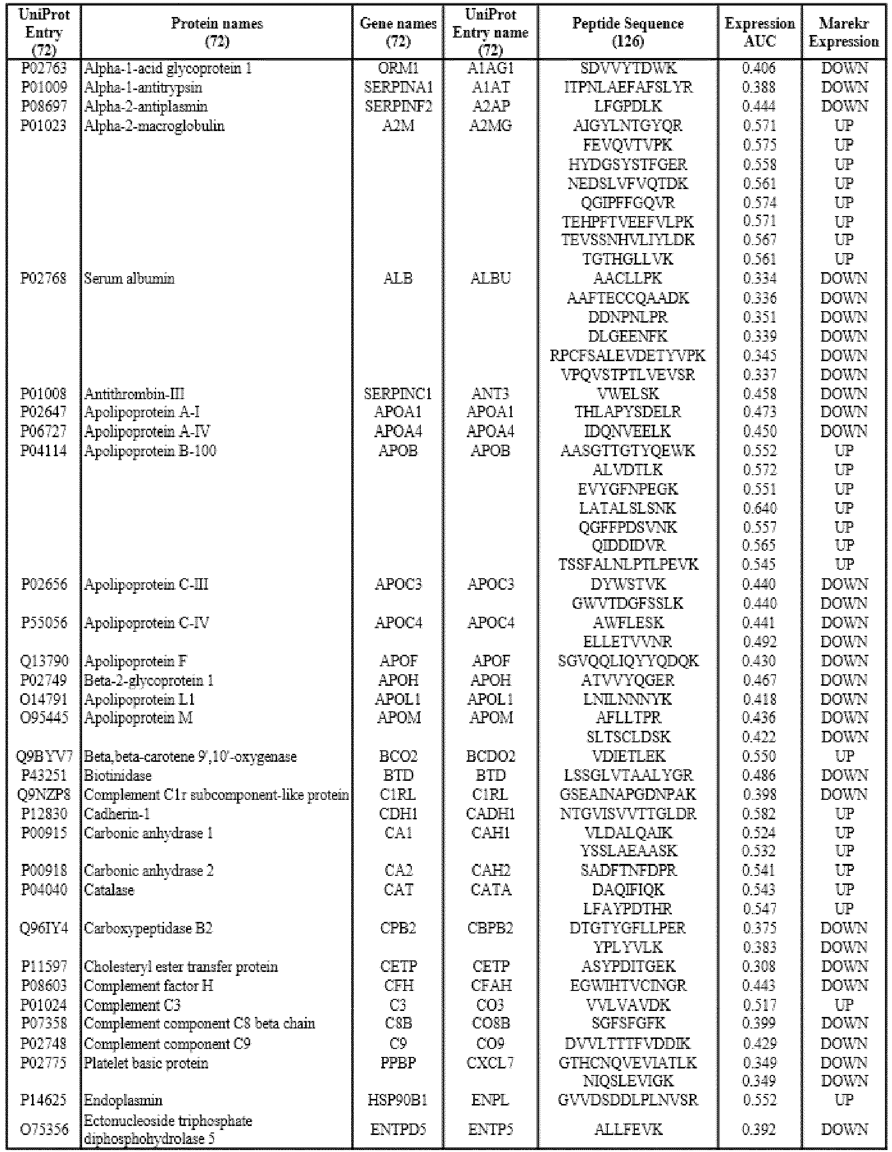

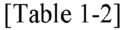

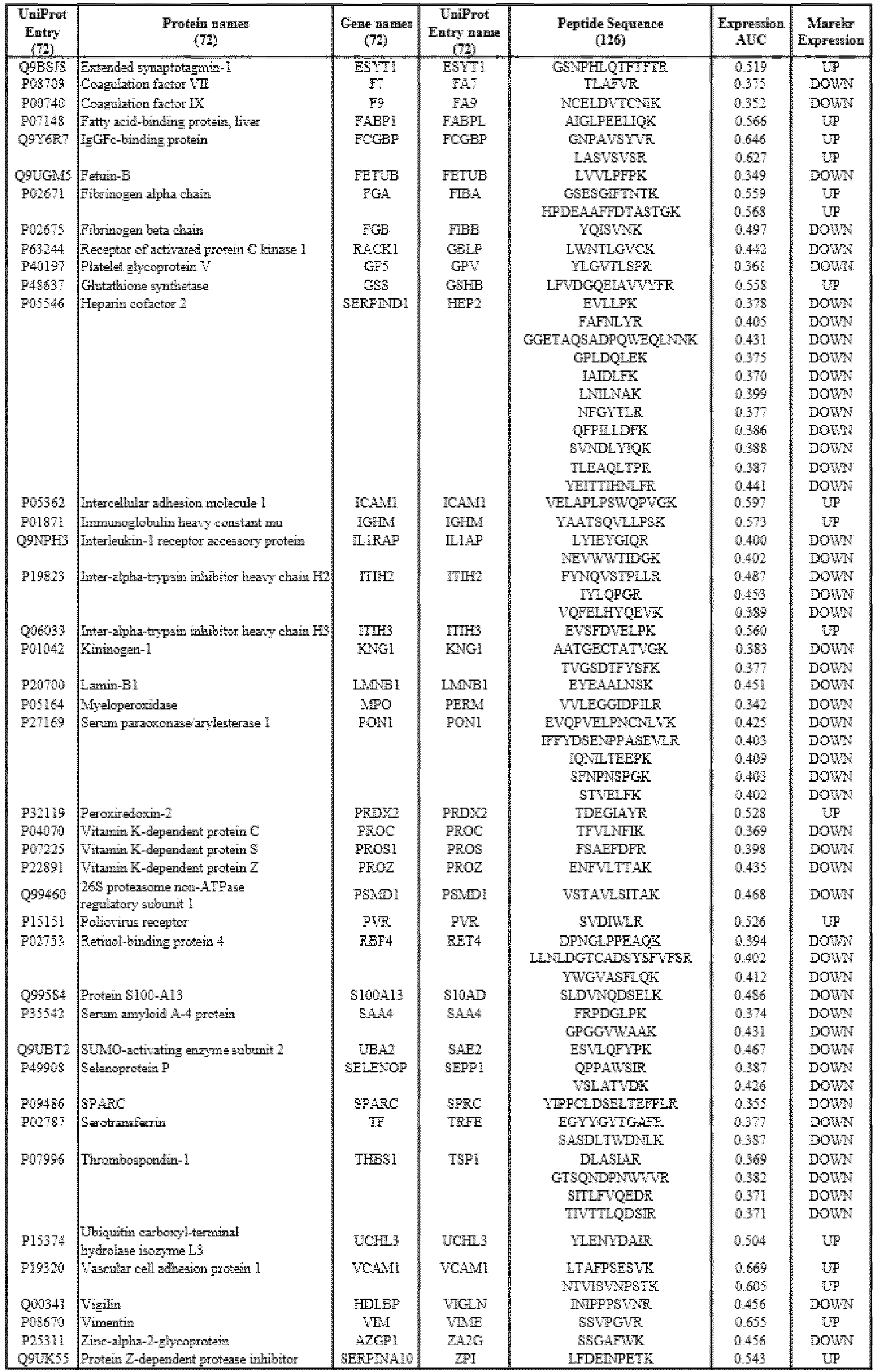

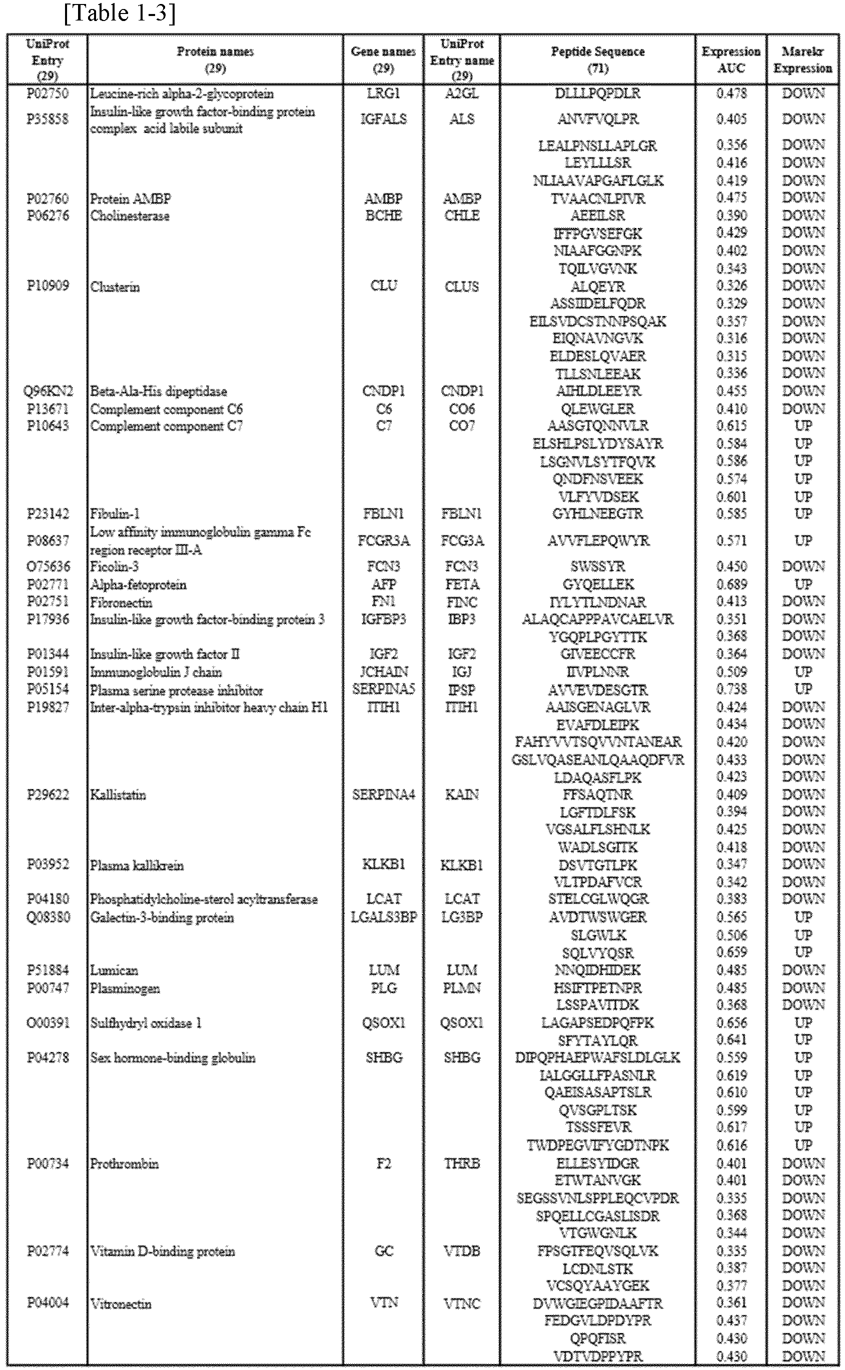

The present markers are useful for the early diagnosis of liver cancer in a subject at high-risk of developing liver cancer.

Liver cancer is a tumor in which hepatocytes lose their own function by continuous various stimuli and transform into cancer cells, which are endowed with endless self-proliferation and spread to surrounding or distant places. It includes a primary cancer and metastatic cancer that occurs in the liver and is migrated to other organs. Primary liver cancer includes hepatocellular carcinoma caused by abnormal liver cells and cholangiocarcinoma caused by abnormal bile duct cells, and vascular sarcoma and more than 90% are hepatocellular carcinoma (HCC).

In one embodiment according to the present disclosure, the liver cancer is a primary malignant tumor occurring in the liver tissue itself, hepatocellular carcinoma. Hepatocellular carcinoma is more common in high risk patients with risk factors such as alcohol abuse, viral hepatitis and metabolic liver disease including liver cirrhosis. Hepatocellular carcinoma has no fibrous stromal and thus has bleeding and cell necrosis. It causes vascular infiltration into the portal system, and in severe cases, hepatic rupture and intraperitoneal blood effusion. The high-risk group of developing liver cancer herein is a patient with hepatitis including viral hepatitis and hepatitis B, and liver disease including liver cirrhosis. These patients are classified as patients having high risk of developing liver cancer in the medical field due to their high incidence of liver cancer.

Screening tests and surveillance tests for diagnosing the progression into liver cancer in high risk group are key for early diagnosis and effective treatment, and the present disclosure provides the markers optimal and effective for these effects and their use.

Hepatitis is an infectious disease that manifests itself as an infection caused by virus particularly hepatitis B virus infection and includes in particular chronic hepatitis. Chronic hepatitis caused by the hepatitis B virus can develop into cirrhosis or liver cancer, and Hepatitis B can also develop to liver cancer.

Liver cirrhosis is a chronic inflammation of the liver that causes regenerative nodule and fibrosis of liver tissue, resulting in hepatocellular damage, scars, decreased liver function, ascites, gastrointestinal bleeding and hepatic coma. Since about 90% of liver cirrhosis patients are diagnosed with liver cancer, early diagnosis of liver cancer is particularly important in high-risk groups.

The marker according to the present disclosure can diagnose the liver cancer early in the high-risk group of developing liver cancer. The classification according to the developmental stage of liver cancer (grade) is known in the art, for example, the Edmondson Steiner grading system based on histological grade of tumor differentiation (Pirisi M et al., Arch Pathol Lab Med. 2010 Dec;134(12):1818-22). Also, American Joint Committee on Cancer (AJCC) TNM Staging for Liver Tumors (7th ed., 2010) or latest edition of Liver Cancer Study Group of Japan (LCSGJ)-T for HCC TNM (Tumor node metastasis) staging may be referred.

According to the references, hepatocellular carcinoma is divided into 1 to 4 stages according to the progression stage, the marker according to the present disclosure can detect stage 1 or precancerous stage of liver cancer. In another embodiment, the early diagnosis includes diagnosis of extremely early stage of liver cancer with less than 5 cm of liver cancer cell tissue.

Precancerous is a condition of premalignant or condition with malignant potential. This is just before the onset of cancer or in the extremely early stages, where the detection of cancer can dramatically improve the efficacy of treatment and survival rate.

Diagnosis and staging of cancer as described above is determined in general according to the type of cancer cells, the degree of differentiation, and/or the degree of metastasis, etc. of the tissue obtained through surgery or biopsy. That is, the treatment and prognosis of solid tumor in general are determined by the TNM stages.

In contrast, in case of liver cancer, TNM staging alone may make it difficult to choose optimal treatment and/or predict prognosis. This is because most liver cancer patients also have chronic liver disease, which limits the selection of optical treatment methods for liver cancer. Unlike other carcinomas, liver cancer can also be diagnosed by imaging tests. Currently, the clinical guidelines for early diagnosis of hepatocellular carcinoma in patients with high-risk including hepatitis B and cirrhosis patients are to visit every six months for ultrasound and AFP test. However, ultrasound examination requires a specialist and the degree of diagnosis varies widely depending on the proficiency of the specialist.

Therefore, by using the markers according to the present disclosure, it is possible for surveillance and/or early diagnosis of the onset of liver cancer, including the liver cancer at extremely early stage, by detecting the marker or combination of the markers and the expression status of the markers in patients with high risk of liver cancer including hepatitis and cirrhosis patients.

In one embodiment according to the present disclosure, the marker or combination of the markers exhibits differential expression with high AUC values in both primary, secondary and tertiary sample groups as described in the Examples herein, but is not limited thereto.

In one embodiment, the markers for early diagnosis of liver cancer in high risk of liver cancer group include at least one marker selected from the group consisting of SERPINA1, ALB, APOB, APOC3, APOC4, APOL1, BTD, CDH1, CA1, CAT, CETP, C3, ESYT1, FCGBP, FETUB, FGA, GP5, GSS, SERPIND1, IL1RAP, ITIH2, LMNB1 MPO, PON1, PRDX2, PVR, SAA4, UBA2, SELENOP, TF, THBS1, UCHL3, HDLBP, AZGP1 and SERPINA10. The markers may further comprise at least one marker selected from the group consisting of LRG1, C7, AFP, JCHAIN, SERPINA5, SERPINA4, LGALS3BP, LUM, QSOX1, SHBG, F2, and VTN.

Further, the markers according to the present disclosure can early diagnose liver cancer in hepatitis patients among the high-risk groups of liver cancer. In one embodiments, these markers are at least one markers selected from the group consisting of ORM1, A2M, ALB, SERPINC1, APOA1, APOA4, APOB, APOC3, APOC4, APOF, APOL1, APOH, APOM, BCO2, CDH1, CPB2, CA1, CA2, CAT, CETP, C8B, PPBP, F9, FABP1, ENTPD5, F7, FCGBP, FETUB, FGA, RACK1, GP5, SERPIND1, ICAM1, IGF2, IL1RAP, ITIH2, ITIH3, KNG1, PON1, PROC, PROS1, PROZ, PSMD1, RBP4, S100A13, SAA4, SELENOP, SPARC, THBS1, VCAM1, VIM and SERPINA1. The markers may further comprise at least one marker selected from the group consisting of IGFALS, BCHE, CLU, CNDP1, C7, FCGR3A, FCN3, AFP, IGFBP3, ITIH1, SERPINA4, KLKB1, LCAT, LGALS3BP, PLG, QSOX1, SHBG, F2, GC and VTN.

Further, the markers according to the present disclosure can early diagnose liver cancer in liver cirrhosis patients among the high-risk groups of liver cancer. In one embodiments, these markers are at least one markers selected from the group consisting of SERPINF2, APOA4, APOB, APOL1, APOM, BCO2,C1RL, CA2, CAT, CETP, CFH, C9, PPBP, HSP90B1, F9, FABP1, FGA, FGB, GSS, SERPIND1, IGHM, ITIH2, MPO, PON1, QSOX1, RBP4, SELENOP, UCHL3, HDLBP, AZGP1 and SERPINA10. The markers may further comprise at least one marker selected from the group consisting of AMBP, BCHE, CLU, C6, C7, FBLN1, AFP, FN1, SERPINA5, SERPINA4, LUM, SHBG, and F2.

The markers according to the present disclosure may be used alone or in combination of at least of two markers, for example 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60. 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 markers, or used in combination with conventional markers. It may also be used in conjunction with existing markers and/or conventional diagnostic methods such as liver ultrasound and the like. Those skilled in the art will be able to select without difficulty the combinations of the markers that meet the desired sensitivity and specificity through methods such as Logistic regression analysis and / or Support Vector Machines with reference to methods described in the Examples herein.

In one embodiment, for comparison of high-risk of liver cancer group (HBV+LC) and liver cancer group, at least one markers selected from SERPINA1, LRG1, ALB, APOB, APOC3, APOC4, APOL1, BTD, CDH1, CA1, CAT, CETP, C3, C7, ESYT1, FCGBP, AFP, FETUB, FGA, GP5, GSS, SERPIND1, JCHAIN, IL1RAP, SERPINA5, ITIH2, SERPINA4, LGALS3BP, LMNB1, LUM, MPO, PON1, PRDX2, PVR, QSOX1, SAA4, UBA2, SELENOP, SHBG, F2, TF, THBS1, UCHL3, HDLBP, VTN, AZGP1, and SERPINA10 may be combined in various combinations. For example, in one embodiment, the combination of the markers was found to have a high diagnostic power with AUC of 0.990 and showed AUC=0.881 in separate independent data set (validation set), indicating good diagnostic power.

In other embodiment, for comparison of high-risk of liver cancer group (HBV+LC) and liver cancer group, at least one markers selected from ALB, APOB, CA1, CAT, CETP, C3, C7, FCGBP, AFP, FETUB, FGA, GP5, SERPIND1, JCHAIN, SERPINA5, ITIH2, SERPINA4, LGALS3BP, MPO, PRDX2, QSOX1, SELENOP, SHBG, TF, THBS1, UCHL3, VTN, AZGP1, and SERPINA10 may be combined in various combinations. For example, in one embodiment, the combination of the markers was found to have a high diagnostic power with AUC of 0.97 and showed AUC=0.928 in separate set (validation set), independent data indicating good diagnostic power.

In one embodiment, the combination of the markers for early diagnosis of liver cancer in high-risk group includes ALB, APOB, APOL1, BTD, CDH1, CAT, CETP, ESYT1, AFP, FGA, GP5, GSS, SERPINA5, ITIH2, ITIH2, SERPINA4, LUM, MPO, PON1, PVR, SAA4, and SERPINA10; APOB, CAT, CETP, C7, AFP, FETUB, FGA, GP5, SERPIND1, SERPINA5, ITIH2, SERPINA4, LGALS3BP, MPO, QSOX1, SHBG, TF, VTN, AZGP1, and SERPINA10; LRG1, APOC3, CETP, AFP, SERPINA5, TF, and SERPINA10; APOC4, AFP, SERPINA5, LMNB1, MPO, PVR, and SERPINA10; SERPINA1, APOB, CAT, CETP, AFP, FETUB, FGA, GSS, SERPINA5, ITIH2, SERPINA4, UBA2, SHBG, F2, THBS1, THBS1, and SERPINA10; CETP, C7, AFP, IL1RAP, SERPINA5, LGALS3BP, MPO, HDLBP, and SERPINA10; ALB, APOB, CA1, CETP, C3, FCGBP, AFP, FETUB, FGA, JCHAIN, SERPINA5,ITIH2, SERPINA4, PRDX2, SELENOP, SHBG, THBS1, UCHL3, and SERPINA10; or ALB, APOB, CA1, CETP, AFP, FETUB, SERPINA5, ITIH2, SERPINA4, MPO, F2, THBS1, and SERPINA10.

Also, the combinations of the present markers can be used for early diagnosis of liver cancer in hepatitis B patients among the high-risk groups of liver cancer.

In one embodiment, for comparison of hepatitis group and liver cancer group, at least one markers selected from ALB, IGFALS, SERPINC1, APOA1, APOA4, APOB, APOC3, APOC4, APOH, APOM, BCO2, CDH1, CA1, CA2, CAT, CETP, BCHE, C7, PPBP, F9, FABP1, FCGBP, FCN3, AFP, FETUB, FGA, IL1RAP, ITIH1, ITIH2, SERPINA4, KNG1, LGALS3BP, PROS1, PROZ, PSMD1, QSOX1, S100A13, VCAM1, and SERPINA10 SERPINA10 may be combined in various combinations. For example, in one embodiment, the combination of the markers was found to have a high diagnostic power with AUC of 0.983 and showed AUC=0.858 in separate independent data set (validation set), indicating good diagnostic power.

In other embodiment, for comparison of hepatitis group and liver cancer group, at least one markers selected from ALB, IGFALS, APOB, APOC3, BCO2, CDH1, CA1, BCHE, C7, PPBP, FABP1, FCGBP, AFP, FETUB, ITIH1, SERPINA4, KNG1, LGALS3BP, PROS1, PSMD1, VCAM1, and SERPINA10 may be combined in various combinations. For example, in one embodiment, the combination of the markers was found to have a high diagnostic power with AUC of 0.970 and showed AUC=0.851 in separate independent data set (validation set) indicating good diagnostic power.

In one embodiment, the combination of these markers includes IGFALS, APOA4, C7, PPBP, F9, FABP1, FCGBP, AFP, ITIH1, SERPINA4, and SERPINA10; IGFALS, APOB, APOC3, PPBP, FABP1, FCGBP, AFP, ITIH1, SERPINA4, PROS1, and SERPINA10; ALB, IGFALS, CETP, C7, PPBP, FABP1, AFP, IL1RAP, ITIH1, ITIH2, SERPINA4, S100A13, and SERPINA10; ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10; IGFALS, CA2, CETP, C7, PPBP, FABP1, FCGBP, FCN3, AFP, FGA, ITIH1, SERPINA4, and SERPINA10; IGFALS, SERPINC1, APOA1, CAT, C7, PPBP, F9, FCGBP, AFP, FGA, ITIH2, SERPINA4, PROZ, QSOX1, and SERPINA10; APOB, APOC4, APOH, CDH1, PPBP, FABP1, AFP, FETUB, SERPINA4, and SERPINA10 or APOB, APOC4, APOH, APOM, CAT, PPBP, FABP1, AFP, FETUB, ITIH1, SERPINA4, and SERPINA10.

Also, the combinations of the present markers can be used for early diagnosis of liver cancer in liver cirrhosis patients among the high-risk groups of liver cancer.

In other embodiment, for comparison of liver cirrhosis group and liver cancer group, at least one markers selected from APOB, APOL1, CAT, CETP, C7, FABP1, AFP, FGA, FN1, IGHM, SERPINA5, ITIH2, SERPINA4, PON1, SERPINA10, BCO2, CFH, BCHE, SELENOP, UCHL3, AZGP1, AMBP, APOM, C1RL, CLU, PPBP, HSP90B1, F9, GSS, RBP4, HDLBP, APOA4, FGB, SERPIND1, LUM, QSOX1, SHBG, C9, FBLN1, SERPINF2, F2, CA2, and C6 may be combined in various combinations. For example, in one embodiment, the combination of the markers was found to have a high diagnostic power with AUC of 0.970 and showed AUC=0.890 in separate independent data set (validation set) indicating good diagnostic power.

In other embodiment, for comparison of liver cirrhosis group and liver cancer group, at least one markers selected from APOA4, APOB, BCO2, C1RL, CAT, CETP, CFH, BCHE, AFP, FGA, FGB, SERPIND1, SERPINA5, SERPINA4, LUM, QSOX1, SELENOP, SHBG, UCHL3, AZGP1, and SERPINA10 may be combined in various combinations. For example, in one embodiment, the combination of the markers was found to have a high diagnostic power with AUC of 0.970 and showed AUC=0.890 in separate independent data set (validation set) indicating good diagnostic power.

In one embodiment, the combination of these markers includes APOB, APOL1, CAT, CETP, C7, FABP1, AFP, FGA, FN1, IGHM, SERPINA5, ITIH2, SERPINA4, PON1, PON1, and SERPINA10; BCO2, CAT, CETP, CFH, BCHE, AFP, FGA, SERPINA5, SELENOP, UCHL3, AZGP1, and SERPINA10; AMBP, APOB, APOM, C1RL, CAT, CETP, CLU, PPBP, HSP90B1, F9, FGA, GSS, SERPINA5, SERPINA4, RBP4, HDLBP, AZGP1, and SERPINA10; APOA4, APOB, C1RL, CETP, AFP, FGB, SERPIND1, SERPIND1, SERPINA5, SERPINA4, LUM, QSOX1, SHBG, and SERPINA10; APOA4, APOB, APOL1, CETP, C9, FBLN1, AFP, FGA, SERPINA5, ITIH2, and SERPINA10; SERPINF2, APOB, CETP, FBLN1, AFP, FGA, FGA, SERPINA5, RBP4, HDLBP, and SERPINA10; APOB, CETP, AFP, FGA, SERPINA5, SERPINA4, F2, and SERPINA10; or APOA4, APOB, APOB, APOL1, CA2, CETP, CLU, C6, AFP, FGA, SERPINA5, ITIH2, SERPINA4, AZGP1, and SERPINA10.

In other embodiment, the at least one markers of the present disclosure may be used in combination with AFP.

The markers according to the present disclosure can be detected to analyze the presence or absence of the markers and/or the expression level, the difference in the expression level or the changes in the expression level at mRNA and/or protein level through various quantitative and/or qualitative analysis methods known in the art.

The term detection or detecting as used herein refers to a determination in quantity, quality and/or changes in expression patterns and/or profiles of the expression. The detection includes a determination of the presence and/or absence as well as the levels of the present markers. The present markers may be detected using the methods known in the art, and the person skilled in the art would be easily able to select appropriate methods for the detection.

The detection of the markers according to the present disclosure may be based on the functional and/or antigenic characteristics thereof.

In one embodiment, the present markers can be detected using the agents that specifically interact with the markers at the nucleic acid level, particularly mRNA level and/or protein level, or the agents that detect the activity or function of the markers may also be used.

For quantitative and qualitative analysis of the markers according to the present disclosure, various known methods for qualitatively or quantitatively detecting proteins or nucleic acids can be used.

The quantitative and qualitative analysis methods at the protein level may include for example a western blot, an ELISA, a radioimmunoassay, an immunodiffusion, an immunoelectrophoresis, an immunostaining, an immunoprecipitation, a complement fixation assay, assay for binding to labelled antibody in solution/suspension, mass spectrometry or protein array employing antibodies and the like.

The quantitative and qualitative analysis methods at the nucleic acid level may include for example a nucleic acid transcription and amplification system, eTag system, a system based on tailed beads, and array system such as nucleic acid array and the like.

These methods are known in the art and may be found for example in chip-based capillary electrophoresis: Colyer et al. 1997. J Chromatogr A. 781(1-2):271-6; mass spectroscopy: Petricoin et al. 2002. Lancet 359: 572-77; eTag systems: Chan-Hui et al. 2004. Clinical Immunology 111:162-174; microparticle-enhanced nephelometric immunoassay: Montagne et al. 1992. Eur J Clin Chem Clin Biochem. 30:217-22 and the like.

In one embodiment of the present disclosure, a mass spectrometry is used for detecting the present markers, which may be performed using the method such as described in the Examples of the present disclosure, or Kim, et al. 2010 J Proteome Res. 9: 689-99; and Anderson, L et al. 2006. Mol Cell Proteomics 5: 573-88 may also be referred.

In one embodiment Multiple Reaction Monitoring (MRM) technology utilizing for example Triple Quadrupole LC-MS/MS and QTRAP and the like, in which, ions are directed to a quadruple anode consisting of four electrode lines, and analyzed according to a mass / charge (m / z) ratio, may be used (refer to FIG. 3). MRM is a method for exactly quantifying multiple markers present in biological samples in minute amount. In MRM, by a first mass filter (Q1) passing through peptide with particular m/z, parent or precursor ions are selected from the ion fragments generated in ionization source and transferred to a collision cell (Q2). And then the precursor ions arrived at the collision cell collide with internal collision gas, and are fragmented into products or daughter ions and transferred to a second mass filter (Q3), from which only the peptide with specific m/z are selected and delivered to a detector which converts them into a digital signal and generates peak chromatograms. In this way, by MRM, only the information of the desired target can be obtained with high selectivity and sensitivity. The peptide to be analyzed is a part of the entire protein sequence to be analyzed, which is used for MRM analysis. An optimal target peptide to be analyzed may be selected from the entire sequences of the protein, which satisfies certain conditions, such as representativeness of the protein to be analyzed, whether it can be modified, a length suitable for MRM analysis, and the like. For example, peptides with 6-30 amino acids in length are selected: if the length is too short, the selectivity is lowered and if the length is too long, the sensitivity is lowered. In addition, methionine, cysteine, and tryptophan are easily chemically modified, such as oxidation, and are thus excluded. In addition, MRM uses an internal standard corresponding to the peptide of interest. The internal standard has the same amino acid sequence as the target peptide described above, but the mass of one or more amino acids constituting the internal standard differs from the amino acid mass of the peptide of interest. However, the amino acid sequence and hydrophobicity are identical to each other and the internal standard peptides elute at the same retention time as the peptides of interest. By this way, it can be confirmed whether the target peptide is actually derived from the target protein.

In one embodiment of the present disclosure, the detection of the marker according to the present application can be carried out through the detection of the corresponding peptides from the marker protein. For example, the corresponding target peptides for each marker are described in Tables 1-1, 1-2 and 1-3. One or more peptides may be used for one protein marker.

In one embodiment of the present disclosure, the at least one amino acids constituting the internal standard material or peptides may be labeled with a radioactive isotopes such as ¹³C or ¹⁵N or mixture thereof (Sable Isotope-Labeled Peptides as Internal Standards peptide; SIS peptides). For example, Gillette et al., 2013, Nature Methods 10:28-34 may be referred. Since the same amount of internal standard is added to all samples, it is possible to normalize the entire analysis result and to verify whether the target peptide to be detected is actually derived from the protein to be analyzed.

In one embodiment, multiple simultaneous quantitative analysis (MRM), used for the detection of markers according to the present invention, uses a mass spectrometry for a target peptide in a sample, detection of specific peptide ions, use of internal standards, and the calculation of the ratio of relative or absolute concentration change between them, and thus is able to quantify the amount of a particular protein or peptide in the physiological state. In the method according to the present invention, MRM analysis may be performed by monitoring Q1 and Q3 values for internal standards and peptides of interest, and may select and use a Q1 / Q3 pair showing the highest signal.

In other embodiment, an immunoassay using sandwich system like ELISA (Enzyme Linked Immuno Sorbent Assay), or RIA (Radio Immuno Assay) and the like may be used for quantitative and/or qualitative detection of the present markers. In this system, the biological samples are reacted with a first antibody fixed to a solid substrate/support such as a glass, a plastic (for example, polystyrene), polysaccharides, a bead, a nylon or nitrocellulose membrane or a microplate well to form a complex and the complex is then allowed to react with an second antibody that is usually labeled with agents that can be detected directly or indirectly such as radioactive substances like ³H or ¹²⁵I, fluorescent materials, chemiluminescent substances, hapten, biotin, or digoxygenin and the like. In some cases, the labeling materials are conjugated with an enzyme such as horseradish peroxidase, alkaline phosphatase, or maleate dehydrogenase that is able to produce colors or color changes or illuminate in the presence of appropriate substrates.

Other methods based on immune reaction may also be used. In other embodiment, an Immuno Electrophoresis such as an Ouchterlony plate, a Western blot, a Crossed IE, a Rocket IE, a Fused Rocket IE, or an Affinity IE, which can detect the markers simply by antigen-antibody reaction may be used. The agents or materials that may be used in the methods described above are known the art and may be found in Enzyme Immunoassay, E. T. Maggio, ed., CRC Press, Boca Raton, Florida, 1980; Gaastra, W., Enzyme-linked immunosorbent assay (ELISA), in Methods in Molecular Biology, Vol. 1, Walker, J.M. ed., Humana Press, NJ, 1984. The intensities of the signals generated by the immunoassay mentioned above are then analyzed, namely compared with the signals from appropriate controls for the determination whether the sample has a disease or not.

The agents or materials used these methods are known in the art for example, a monoclonal antibody, a polyclonal antibody, a substrate, an aptamer, an avimer, a peptidomimetic, a receptor, a ligand and a cofactor that are specifically interact with the present markers may be used. The agents or materials that bind or interact specifically with the markers of the present disclosure can be utilized by means of chip or with nanoparticles.

The detecting agents may also be labeled for the direct or indirect signal detection via sandwich forms. In the case of direct labeling method, biological samples such as serum to be used for array may be labeled with a fluorescent material such as Cy3 and Cy5. In the case of indirect labeling, unlabeled samples are allowed to bind to the detecting agent which is attached to the array, and then the target or marker proteins are detected by the labeled antibodies which specifically recognize the marker or target. In the case of sandwich methods, the sensitivity and specificity of the detection is usually high and able to detect the marker at the pg/mL level. In addition, labeling agents including such as radioactive materials, agents that produce visible colors under proper condition, magnetic particles and high-density electron particle and the like may also be used. For the detection of fluorescence signals, a scanning confocal microscopy may be used, which are available from for example, Affymetrix, Inc. or Agilent Technologies, Inc. and the like.

The compositions of the present disclosure may also include one or more additional components as needed for the detection of the present marker(s), which include, for example, binding buffers, reagents for preparing biological samples, syringes for blood collecting or negative and/or positive controls.

The compositions of the present disclosure in which various agents as described above may be contained may be provided for an ELISA, a dip stick rapid kit, a MRM, microarray, a gene amplification, or an immunoassay. The reagents which may be included for a particular kit/composition may be appropriately selected from the known agents in view of the present disclosure.

In one embodiment, an ELISA or a dip stick rapid kit is used, and in this case, the antibodies to detect the present markers may be provided as conjugated to a substrate/support, for example to wells of a multi-well plate or surface of a glass slide or a nitrocellulose paper. A dip stick is a form widely used in POCT (point of care technology) in which the biomarkers of the present disclosure may be detected by employing one or more antibodies that are conjugated to a substrate such as nitrocellulose paper which is then contacted with a sample such as serum for example by dipping one end of the stick to the serum and then the sample is moved through the substrate by a capillary action, and the markers are detected by a color that is developed when the markers of interest are bound by the antibody attached to the substrate.

In other embodiment, an MRM kit which is based on peptide analysis is provided. MRM analysis may be performed or used as described above. The MRM is a method using the peptides that selectively recognize a particular protein, and can detect the markers in biological samples in a more stable manner compared to the existing methods that employ antibodies that are sensitive to environmental conditions such as temperature and humidity and the like. For example, the peptide which may be employed are listed in Table 1-1, 1-2 and 1-3 and one or more peptides per each marker may be used.

In other aspect, the detecting agents which may be used for the present disclosure may be provided as a type of array such as microarray or chip. The detecting agents may be attached to the surface of a substrate such as glasses or nitrocellulose. The references for the array preparation technology may be found in for example, Schena et al., 1996, Proc Natl Acad Sci USA. 93(20): 10614-9; Schena et al., 1995, Science 270(5235):467-70; and U.S. Pat. Nos. 5,599,695, 5,556,752 and 5,631,734. The detecting agents that can be provided as attached to an array include, but are not limited to, antibodies specifically recognizing the present marker, or antibody fragments, or aptamers, avimers (avidity multimer) or peptidomimetics.

In other aspect, the present disclosure is related to methods of early diagnosis or surveillance of liver cancer of a subject with high-risk of developing cancer using the marker or the combinations of the markers disclosed herein.

In still other aspect, the present disclosure is related to methods of detecting the marker or the combinations of the markers disclosed herein for early diagnosis or surveillance of liver cancer of a subject with high-risk of developing cancer. These methods can be particularly performed in vitro.

In still other aspect, the present disclosure is related to methods of detecting the marker or the combinations of the markers disclosed herein to provide information for early diagnosis or surveillance of liver cancer of a subject with high-risk of developing cancer.

Reference may be made to the markers and combinations of the markers employed in the various methods according to the present disclosure as described above.

In one embodiment of the present methods, for early diagnosis or surveillance of liver cancer of a subject with high-risk of developing cancer, the present method comprises a step of detecting in the blood sample from a subject in need thereof the presence or absence and/or concentration of nucleic acids and/or proteins of one or more biomarkers selected from the group consisting of ORM1 (Alpha-1-acid glycoprotein 1), SERPINA1 (Alpha-1-antitrypsin), SERPINF2 (Alpha-2-antiplasmin), A2M (Alpha-2-macroglobulin), ALB (Serum albumin), SERPINC1 (Antithrombin-III), APOA1 (Apolipoprotein A-I), APOA4 (Apolipoprotein A-IV), APOB (Apolipoprotein B-100), APOC3 (Apolipoprotein C-III), APOC4 (Apolipoprotein C-IV), APOF (Apolipoprotein F), APOH (Beta-2-glycoprotein 1), APOL1 (Apolipoprotein L1), APOM (Apolipoprotein M), BCO2 (Beta,beta-carotene 9',10'-oxygenase), BTD (Biotinidase), C1RL (Complement C1r subcomponent-like protein), CDH1 (Cadherin-1), CA1 (Carbonic anhydrase 1), CA2 (Carbonic anhydrase 2), CAT (Catalase), CPB2 (Carboxypeptidase B2), CETP (Cholesteryl ester transfer protein), CFH (Complement factor H), C3 (Complement C3), C8B (Complement component C8 beta chain), C9 (Complement component C9), PPBP (Platelet basic protein), HSP90B1 (Endoplasmin), ENTPD5 (Ectonucleoside triphosphate diphosphohydrolase 5), ESYT1 (Extended synaptotagmin-1), F7 (Coagulation factor VII), F9 (Coagulation factor IX), FABP1 (Fatty acid-binding protein, liver), FCGBP (IgGFc-binding protein), FETUB (Fetuin-B), FGA (Fibrinogen alpha chain), FGB (Fibrinogen beta chain), RACK1 (Receptor of activated protein C kinase 1), GP5 (Platelet glycoprotein V), GSS (Glutathione synthetase), SERPIND1 (Heparin cofactor 2), ICAM1 (Intercellular adhesion molecule 1), IGHM (Immunoglobulin heavy constant mu), IL1RAP (Interleukin-1 receptor accessory protein), ITIH2 (Inter-alpha-trypsin inhibitor heavy chain H2), ITIH3 (Inter-alpha-trypsin inhibitor heavy chain H3), KNG1 (Kininogen-1), LMNB1 (Lamin-B1), MPO (Myeloperoxidase), PON1 (Serum paraoxonase/arylesterase 1), PRDX2 (Peroxiredoxin-2), PROC (Vitamin K-dependent protein C), PROS1 (Vitamin K-dependent protein S), PROZ (Vitamin K-dependent protein Z), PSMD1 (26S proteasome non-ATPase regulatory subunit 1), PVR (Poliovirus receptor), RBP4 (Retinol-binding protein 4), S100A13 (Protein S100-A13), SAA4 (Serum amyloid A-4 protein), UBA2 (SUMO-activating enzyme subunit 2), SELENOP (Selenoprotein P), SPARC (SPARC), TF (Serotransferrin), THBS1 (Thrombospondin-1), UCHL3 (Ubiquitin carboxyl-terminal hydrolase isozyme L3), VCAM1 (Vascular cell adhesion protein 1), HDLBP (Vigilin), VIM (Vimentin), AZGP1 (Zinc-alpha-2-glycoprotein) and SERPINA10 (Protein Z-dependent protease inhibitor). LRG1 (Leucine-rich alpha-2-glycoprotein), IGFALS (Insulin-like growth factor-binding protein complex acid labile subunit), AMBP (Protein AMBP [Cleaved into: Alpha-1-microglobulin), BCHE (Cholinesterase), CLU (Clusterin), CNDP1 (Beta-Ala-His dipeptidase), C6 (Complement component C6), C7 (Complement component C7), FBLN1 (Fibulin-1), FCGR3A (Low affinity immunoglobulin gamma Fc region receptor III-A), FCN3 (Ficolin-3), AFP (Alpha-fetoprotein), FN1 (Fibronectin), IGFBP3 (Insulin-like growth factor-binding protein 3), IGF2 (Insulin-like growth factor II), JCHAIN (Immunoglobulin J chain), SERPINA5 (Plasma serine protease inhibitor), ITIH1 (Inter-alpha-trypsin inhibitor heavy chain H1), SERPINA4 (Kallistatin), KLKB1 (Plasma kallikrein), LCAT (Phosphatidylcholine-sterol acyltransferase), LGALS3BP (Galectin-3-binding protein), LUM (Lumican), PLG (Plasminogen), QSOX1 (Sulfhydryl oxidase 1), SHBG (Sex hormone-binding globulin), F2 (Prothrombin), GC (Vitamin D-binding protein) and VTN (Vitronectin); comparing the results of the detection of the presence or absence or amount of the nucleic acids or protein to those of a corresponding marker of control sample; and determining the subject has a liver cancer wherein the change in the amount and/or in the presence or absence of the biomarker at the nucleic acid and/or protein level in the subject from the control sample indicates that the subject has liver cancer.

In one embodiment, the markers are selected from the group consisting of ORM1, SERPINA1, SERPINF2, A2M, ALB, SERPINC1, APOA1, APOA4, APOB, APOC3, APOC4, APOF, APOH, APOL1, APOM, BCO2, BTD, C1RL, CDH1, CA1, CA2, CAT, CPB2, CETP, CFH, C3 (Complement C3), C8B, C9, PPBP, HSP90B 1, ENTPD5, ESYT1, F7, F9, FABP1, FCGBP, FETUB, FGA, FGB, RACK1, GP5, GSS, SERPIND1, ICAM1, IGHM, IL1RAP, ITIH2, ITIH3, KNG1, LMNB1, MPO, PON1, PRDX2, PROC, PROS1, PROZ, PSMD1, PVR, RBP4, S100A13, SAA4, UBA2, SELENOP, SPARC, TF, THBS1, UCHL3, VCAM1, HDLBP, VIM, AZGP1 and SERPINA10. In other embodiment, the markers may further comprise at least one markers selected from the group consisting of LRG1, IGFALS, AMBP, BCHE, CLU, CNDP1, C6, C7, FBLN1, FCGR3A, FCN3, AFP, FN1, IGFBP3, IGF2, JCHAIN, SERPINA5, ITIH1, SERPINA4, KLKB1, LCAT, LGALS3BP, LUM, PLG, QSOX1, SHBG, F2, GC and VTN.

The method according to the present invention may also be used in one embodiment for surveillance or early diagnosis of liver cancer onset among high risk groups of liver cancer, in particular cirrhosis patients, or in other embodiments, for surveillance of liver cancer development or early diagnosis of liver cancer onset, especially for hepatitis B patients. For the markers, reference may be made to the foregoing.

Markers used in the method according to the invention can also be used in combination of two or more, with reference to the foregoing.

In the method according to the invention, the method of detecting or quantifying one or more markers can be referred to the foregoing, and methods such as mass spectrometry, in particular MRM analysis, microarrays, especially protein microarrays, nucleic acid amplification, for example nucleic acid amplification after synthesizing mRNA with cDNA or various immunoassay methods such as ELISA can be used.

In one embodiment, the method according to the present disclosure is carried out by MRM analysis. For the methods of MRM, reference may be made to the foregoing. The peptides of each markers used for the MRM are listed in Table 1-1, 1-2 and 1-3, and when bloods are used as samples, the proteins in the sample are depleted and degraded and treated with proteinase (for example trypsin). For example, in the method according to the present invention, the peak area is derived from MRM analysis of the target peptide using software such as Skyline, and then normalized to the analysis result of the internal standard to detect the amount of the present markers in the sample. The amount of marker along can be detected. The peak area of the target peptide of the sample are divided by the peak area of the standard internal peptide to calculate the amount of the present marker.

The control samples which are employed in the present methods are samples from Hepatitis B and/or liver cirrhosis patients. Thus, early diagnosis of liver cancer using a marker according to the present application can diagnose patients progressing from hepatitis B or liver cirrhosis to liver cancer, which is advantageous for surveillance and especially early diagnosis.

In the step of determining whether the subject has cancer in the present methods, the changes in the expression level, increase or decrease depending on the particular markers, of the present markers in comparison to the control as described above indicate that the subject has an early stage of liver cancer.

In the markers according to the present invention, the markers of which expression are increased in early stage of liver cancer in comparison with the control are as follows: IGHM, C3, HSP90B1, JCHAIN, ESYT1, GSS, CAT, UCHL3, PVR, APOB, FBLN1, CA1, ITIH3, PRDX2, FCGR3A, QSOX1, FABP1, CA2, FGA, LGALS3BP, CDH1, BCO2, ICAM1, A2M, C7, VIM, SERPINA10, FCGBP, AFP, SHBG, VCAM1 and SERPINA5.

In the markers according to the present invention, the markers of which expression are decreased in early stage of liver cancer in comparison with the control are as follows: CLU, CPB2, KLKB1, GP5, PROC, THBS1, RBP4, IGFBP3, PPBP, GC, FCN3, BCHE, ENTPD5, ALB, SERPIND1, PROS1, SPARC, F9, KNG1, FGB, MPO, CETP, PLG, SERPINF2, LRG1, C8B, LCAT, IGF2, FETUB, FN1, APOH, SAA4, SELENOP, SERPINA4, F7, TF, ORM1, RACK1, ITIH1, C6, IGFALS, APOC4, APOM, LUM, APOL1, FGA, AZGP1, IL1RAP, LMNB1, VTN, CNDP1, SERPINC1, PON1, APOC3, PROZ, C1RL, CFH, APOF, APOA4, C9, SERPINA1, UBA2, ITIH2, APOA1, BTD, HDLBP, AMBP, S100A13, PSMD1 and F2.

Biological samples or samples that can be used in the compositions, kits or methods according to the present invention are blood samples such as whole blood, serum or plasma. In one embodiment herein the serum sample in particular is used.

Biological samples that can be used in the compositions, kits or methods according to the present invention are obtained from mammals, particularly humans. Human subjects include those who develop hepatitis, cirrhosis, or cirrhosis and need to monitor/surveillance of liver cancer development and early diagnose liver cancer. In other embodiments, the method may be performed to determine whether liver cancer is present in a subject having symptoms related to a liver disease such as abdominal pain, hypertrophy, ascites, jaundice, muscle weakness, or esophageal varices.

When two or more markers of the present disclosure are used as combination, a dataset may be generated that includes a profile, i.e., quantitative information related to the expression of the marker proteins in a sample. After obtaining a profile of the markers tested, a comparison of the results with a reference group or a control group determines whether a subject's sample develops liver cancer.

Methods known in the art can be used to compare marker profiles between control groups and test groups using samples. For example, digital image comparison of expression profiles, comparisons of expression data using DB, or US 6,308,170 and 6,228,575 may be referred. Profiles obtained through detecting the marker according to the present disclosure can be processed using data analysis methods known in the related art such as for example, nearest neighbor classifier, partial-least squares, SVM, AdaBoost and clustering-based classification methods. For example, Ben-Dor et al (2007, J. Comput. Biol. 7: 559-83), Nguyen et al (2002, Bioinformatics 18:39-50), Wang et al (2003, BMC Bioinformatics 4:60), Liu et al (2001, Genome Inform. Ser. Workshop Genome Inform.12:14-23), Yeang et al (2001, Bioinformatics 17 Suppl 1: S316-22) and Xiong (2000, Biotechniques 29(6): 1264-8, 1270) and the like may be referred.

In addition, various statistical processing methods may be used to determine that the quantification result obtained by detecting the marker of the present application is significant to discriminate early liver cancer. In one embodiment, logic regression method is used as a statistical processing method and Ruczinski, 2003, Journal of Computational and Graphical Statistics 12:475-512 may be referred. Other analysis methods which may be used in the present disclosure may includes nearest shrunken centroids (Tibshirani. 2002 PNAS. 99:6567-72), random forests (Breiman. 2001. Machine Learning 45:5-32 and MART (Hastie. 2001. The Elements of Statistical Learning, Springer).

In one embodiment, statistical processing may determine the level of confidence in the significant difference between the results obtained in the subject-derived sample and the control group for diagnosis of liver cancer. The raw data used for statistical processing are the values analyzed in duplicate, triple or multiple for each marker.

In another embodiment, the method may be performed several times over a period of time, for example over a year, and may be used to monitor trends in expression patterns. The increase or decrease in the expression of the marker can be monitored to correlate with early liver cancer if there is a change. It can be used to determine early diagnosis, progression, exacerbation, or alleviation of liver cancer, compared to previous test values for the same subject or the value of the control group. Based on changes in the marker expression over time, preventive measures can be taken to prevent progression to liver cancer or to severe liver cancer.

In conjunction with or after the use of the method according to the present invention, for the confirmation of liver cancer, the method according to the present invention may be used, for example, accompanied by conventional blood marker (AFP) examination, ultrasound, computerized axial tomography (CT scan) or magnetic resonance imaging (MRI).

In one embodiment, when the subject is determined to have early stage of liver cancer using the markers according to the present application, the cancer can be confirmed through a detailed examination such as MRI and/or PET-CT, and appropriate treatment can be performed.

The present disclosure is further explained in more detail with reference to the following examples. These examples, however, should not be interpreted as limiting the scope of the present invention in any manner.

### EXMAPLES

### EXAMPLE 1: The information of the clinical samples used in the present disclosure

The serum samples from a total of 440 patients were used for the analysis and the patients include patients confirmed to have liver cancer and patients at high risk of developing liver cancer (hepatitis B and liver cirrhosis patients) approved by the Institutional Review Board of Seoul Asan Medical Center (IRB approval No. 2015-1156). The serum was collected by centrifugation for 10 min at 4°C. and 3000 rpm immediately after taking blood samples from each patient. Serum was stored at 80 °C. in 50 µl aliquots until use for the analysis.

Both the patient group having a high-risk of developing liver cancer and the patient group confirmed to have liver cancer diagnosed in ASAN medical center were over 20 years of age and confirmed to have a good liver function (Child-Pugh class A). In the case of the liver cancer patients, only the patients at an extremely early stage of liver cancer with tumor less than 5 cm in size determined by imaging examination (CT or MRI) or biopsies were selected. For the patients at high-risk of developing liver cancer, only the patients diagnosed with chronic hepatitis B or liver cirrhosis without the history of liver cancer determined by imaging examination or blood test were selected. A total of 440 serum samples were received over three incidences. One hundred fifty samples were first received and includes samples from 50 hepatitis B patients, 50 liver cirrhosis patients and 50 liver cancer patients. The second batch of a total of 150 sample include samples from 50 hepatitis B patients, 50 liver cirrhosis patients and 50 liver cancer patients. The third batch of a total of 140 samples includes samples from 57 hepatitis B patients, 43 liver cirrhosis patients and 40 liver cancer patients. The clinical information of the patients from whom the samples were collected are disclosed in Table 2.

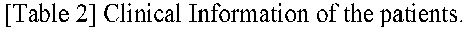

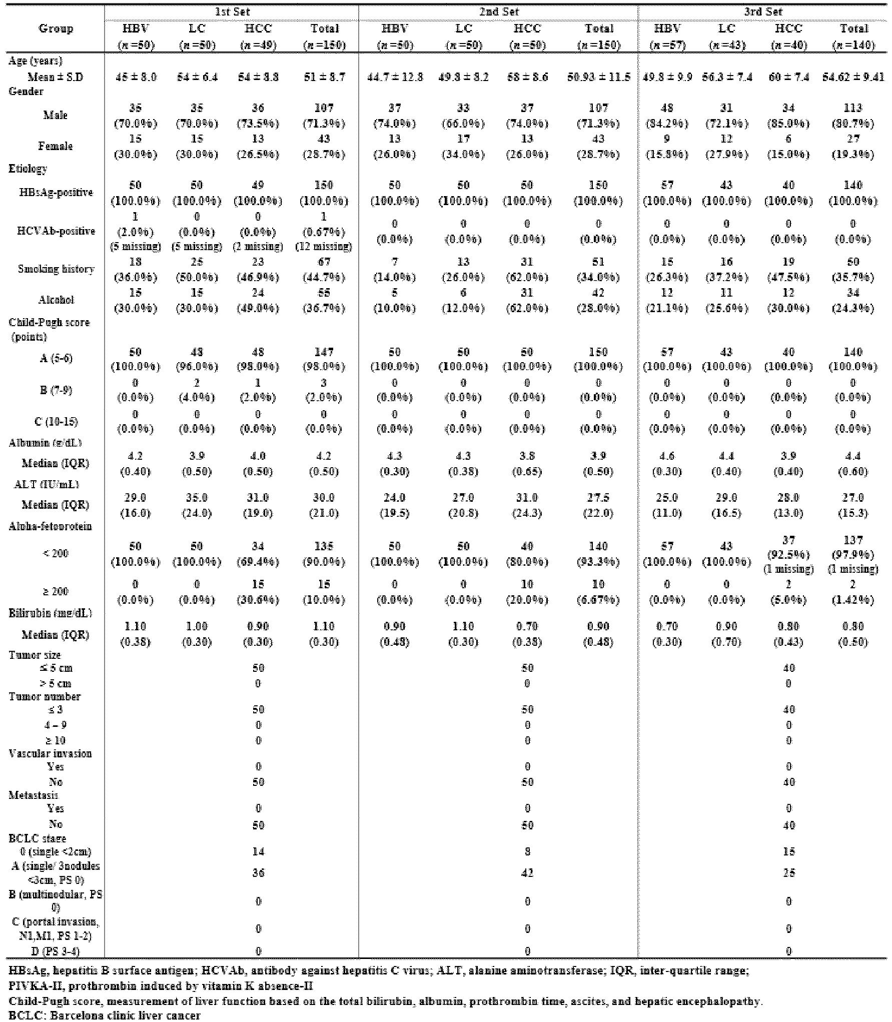

### EXAMPLE 2. Screening the biomarker

### EXAMPLE 2-1: Selection of target marker candidates

1,152 proteins from the LiverAtlas database, and 59 proteins from Human Protein Atals, which are currently the most comprehensive resource associated with liver disease, have been selected. And also 1, 304 proteins were selected by proteome profiling using 6 pairs (normal=6, HCC=6) of tissues. Further 28 proteins approved by Food and Drug Administration (FDA) or Laboratory Developed Test (LDT) and 1 protein maker for diagnosis of liver cancer previously developed were included. Thus, a total of 2189 proteins were selected. Among 2,189 proteins, only the secretory proteins for the quantitative analysis using blood were selected using 8 prediction tools: Plasma proteome database, TargetP, SignalP, SecretomeP, GO annotation, ExoCarta, TMHMM prediction, Uniprot DB). After then the candidates were reviewed in bioinformatical analysis by curator or papers using UniprotKB database and a total of 1,738 candidates were selected. Then Public spectral library (ISB, NIST, SWATHAtlas) and homemade serum/tissue spectrum library were used to select a final candidate that have peptide MS/MS data and thus can be analyzed by Mass spectrometry, resulting in a total of 1,693 proteins and 23,184 peptides derived therefrom as a final candidate.

### EXAMPLE 2-2: Screening detectable marker target candidates

Then out of 1,693 candidate proteins and 23,184 peptides therefrom as described in Example 2-1, the proteins that have technical reproducibility as well as the expression pattern that is different among liver cirrhosis group, hepatitis group and liver cancer group were selected.

For this, the samples from the first batch were pooled by each group of 50 hepatic cancer patients, 50 cirrhosis patients, and 50 hepatitis B patients, respectively. Then the samples prepared were pre-treated and made into peptides and used for MRM analysis using 6490-QqQ Mass spectrometer of Agilent Inc as described in Example 3. The MRM analysis were performed 3 times for each set.

The criteria for selecting the peptides as a detectable target is as follows: whether the peak is detected within 5 minutes before and after the expected Retention time; whether 3 or more transition is observed in the same RT; and whether a dot product (dotp) score, indicating the probability that the detected peak is indeed the predicted protein is 0.6 or higher. Based on the above criteria, a total of 681 proteins and a total of 1,583 peptides were selected.

### EXAMPLE 2-3: Screening quantifiable marker target candidates

Semi-Quantitative MRM analyses using each sample of 681 proteins and 1,583 peptides derived therefrom selected as in Example 2-2 were performed to select quantifiable marker candidates for surveillance or monitoring/diagnosis of high-risk group of developing liver cancer by using MRM as a platform.

For this, random numbers were generated for a total of 150 samples from 50 patients with HCC, 50 patients with cirrhosis (LC), and 50 patients with hepatitis B (HBV), and divided into 10 batches of 15 samples each. Further each batch was adjusted to have the similar percentage of samples from each group.

Semi-quantitative MRM analyses were performed on 150 serum samples to filter targets to be synthesized in high purity. Semi-quantitative) MRM analyses were performed by using iRT (Indexed retention time) Kit (Biognosys Inc) which consists of 11 non-naturally occurring synthetic peptides. By performing the following comparison experiments: Comparison of Hepatitis B Group (HBV) and Liver cancer group (HCC)(HBV vs. HCC); Comparison of Liver Cirrhosis Group (LC) and Liver cancer group (HCC) (LC vs. HCC); Comparison of Hepatitis B Group (HBV) and Liver Cirrhosis Group (LC) (HBV vs. LC); and Comparison of Group of high-risk of developing cancer (Hepatitis B and Liver Cirrhosis, HBV+LC) and Liver Cancer Group (HCC) (HBV+LC vs. HCC), the peptides having AUC ≥ 0.65 and some peptides having AUC less than 0.65 but ≥ 0.60 were selected resulting in 276 proteins and 542 peptides.

Next, SIS (Stable Isotope-labeled Synthetic) peptides (used as an internal standard, peptides corresponding to the endogenous peptide labeled with stable radioisotope) corresponding to each of the above 276 proteins and 542 peptides were used to confirm again that the selected proteins and peptides are actually present in blood using MRM assay as described above on each sample pooled from 50 liver cancer patients, 50 liver cirrhosis patients and 50 hepatitis patients, respectively. All the peak areas of the target peptides from MRM assay were divided by the corresponding peak area of the internal standard peptide for normalization. 50-fmol of the SIS peptide were injected to detect the corresponding endogenous peptide. As a result, 181 proteins and 421 peptides were detected.

Then, to check the signal interference by the peptides other than the target endogenous peptides in the complex blood sample in the MRM assay, for the above 181 proteins and 421 peptides, the product ion (Q3) intensity pattern of the SIS peptides and the blood endogenous peptides were obtained 3 times from each sample pooled from 50 liver cancer patients, 50 liver cirrhosis patients and 50 hepatitis patients, respectively. To determine whether the signal interference is present, AuDIT (Automated detection of inaccurate and imprecise transitions) is used to compare the relative intensity of product ion of SIS peptide and the endogenous peptide in blood (P-value threshold : 0.05), and then it was confirmed whether the peak area of the peptides in the blood and the SIS peptide were constantly detected in the repeated measurements (CV threshold: 0.2). As a result, a total of 177 endogenous proteins and 385 peptides derived therefrom, which did not show any signal interference between the target peptides and thus rendering accurate quantification, were selected as final marker candidates.

### EXMAPLE 3: MRM Analysis

### EXMAPLE 3-1: Pretreatment of clinical serum sample and digestion of serum proteins to make peptides.

To discover markers in the blood found in low concentrations, high abundant six proteins, i.e., albumin, IgG, IgA, haptoglobin, transferrin, alpha-1-antitrypsin in the blood were depleted. Through the depletion process, about 85% by mass of the total proteins present in the blood was removed, and the proteins corresponding to the rest 15% were used for the next analysis. The depletion was performed using MARS (Multiple affinity removal system, Agilent, USA) column according to the manufacturer's instruction.

The serum samples treated with the depletion process were concentrated and the concentration was measured by BCA (Bicinchoninic acid) method. For this, 100 µg of serum sample was treated with 0.2% Rapigest (waters)/20 mM DTT/100 nM ABC Buffer (Tris pH 8.0), which was then incubated at 60°C for 60 min. Then the samples were treated with IAA at the final concentration of 100 mM and incubated for 30min at RT. Then the sample was treated with trypsin at the concentration ratio of 1:50 of trypsin and serum. Then the samples were treated with 10% FA and incubated at 37°C for 1hr, and then centrifuged at 15,000 rpm, 4°C for 1hr to obtain the supernatants.

### EXAMPLE 3-2: Internal standard peptides

The SIS peptide (stable-isotope labeled standard) used as internal standard peptides labelled with heavy isotope were synthesized from JPT Inc (Germany). In the SIS peptides, ¹²C and ¹⁴N of Lysin (Lys, K) or Arginine (Arg, R) at the C-terminus of the peptides were substituted with ¹³C and ¹⁵N, respectively. The SIS peptides are different from its corresponding endogenous peptides in the mass, but have the same amino acid sequence and hydrophobicity with the corresponding endogenous peptide, thus they are eluted at the same RT on the chromatogram. SIS peptides were used by adding them to the serum peptide sample at the concentration of 50fmol for the normalization and confirmation of the endogenous peptide detection.

### EXAMPE 3-3: Quantification using MRM analysis

1260 Capillary LC system (Agilent Technologies, Santa Clara, CA) having two pumps coupled to 6490 triple quadrupole (QqQ) mass spectrometer of Agilent with Jetstream electrospray source was used for all the MRM-MS analysis.

Liquid chromatography was used to perform desalting and peptide separation by mobile phase A [water 0.1% (v / v) formic acid] and mobile phase B [acetonitrile 0.1% (v / v) formic acid]. 10.0µL of the samples to be analyzed was injected into the analytical column by passing through the guard column. Flow rate was used at 40µL / min. The injected sample was passed through a guard column (Agilent Zorbax StableBond C18) (30 mm × 2.1, 1.8 µm, 600 bar) and desalted and first (ID) fractionated. The first fractionated peptides were 2nd (2D) fractionated and eluted by passing through the Analytical column (Agilent Zorbax Eclipse SB-C18) (150mm × 0.5mm, 3.5µm) by mobile phase A and mobile phase B. Peptides were eluted with a mobile phase B (0.1% formic acid in acetonitrile) for 5 min, 10% to 60%, and 90% concentration gradient for 1 minute. Mass spectrometry was used to monitor in MRM mode the transition of the eluted peptides. The gas temperature was 250 ° C, the gas flow was used at 15 L / min, the nebulizer was 30 psi, the sheath gas temperature was 350 ° C, and the sheath gas flow was used at 12 L / min. Capillary voltage and nozzle voltage were used at 2500 V and 2000 V, respectively. The resolution in Quadruple 1 (Q1) and Quadruple 3 (Q3) is set in units. The dwell time was set to have a total cycle time of about 2 sec so that the retention time eluted is selected after all the peptides were analyzed. Based on this, the analysis was performed by scheduled MRM method with window size 5 min.

### EXAMPLE 3-4: MRM Data Analysis.

The data obtained in Example 3-3 were used to select the peptide and fragment ions for MRM analysis using Skyline (http://proteome.gs.washington.edu/software/skyline). Skyline is an open source software for MRM method development and analysis (Stergachis AB, et al., 2011, Nat Methods 8: 1041-1043). The MRM analysis raw data file produced by the mass spectrometer was imported into the skyline program and aligned to quantify the features. The Savitzky-Golay method was applied to smooth the data points. The MRM-MS analysis results were visually examined to calculate the peak area of the transition. Relative abundances were compared using peak areas normalized to internal standard peptides (SIS peptides).

### EXAMPLE 3-5: Statistical Analysis

In order to identify biomarkers with different expression levels/patterns in high-risk of developing liver cancer and hepatocellular carcinoma groups. Univariable analysis and multivariable analysis were performed to evaluate the markers on the following groups each consisting of a comparison and control group: High risk group (HBV+LC) vs. Liver cancer group (HCC); Hepatitis B group (HBV) vs. Liver cancer group (HCC); Liver cirrhosis group (LC) vs. Liver cancer group (HCC).

Firstly, for univariable analysis, 385 peptides from 177 protein candidates of the markers were analyzed 1:1 in the three groups above by logistic regression. The analyses were performed on each of the three experiments. The analysis results were evaluated for the efficacy of the present markers by using ROC (Receiver Operator Characteristic). The ROC curve represents how the sensitivity and specificity changes in what relationship on a two-dimensional plane. The larger the AUC (Area Under Curve) value corresponding to the area under the ROC, the better the diagnostic method. The markers with AUC ≧ 0.60 were selected herein as markers showing differences in the expression in the comparison-control group.

Secondly, for multivariable analysis, 385 peptides from 177 protein candidates of the markers were analyzed in the three groups above. The first and second experiments were integrated and used as analytical data, and the results of the third experiment were used to verify the results of the multivariate analysis. As analytical methods, logistic regression and support vector machine were used. Two kinds of resampling methods were used in reference to prior art. The cross-validation method was set to 5-fold and 10-fold for analysis referring to Kim H. et al., Sci Rep. 2017 Aug 25; 7 (1): 9449 and Kim H. et al., Mol Cell Proteomics. 2017 Jul; 16 (7): 1312-1323 was analyzed by setting 5-fold and 10-fold. Prior Art Meyers RL et. al., Lancet Oncol. 2017 Jan; The bootstrap method was analyzed by dividing the analysis data into ratios of 0.7 and 0.9 by referring to Meyers RL et. al., Lancet Oncol. 2017 Jan; 18 (1). The ROC curve and the accuracy were used to confirm the diagnostic power and accuracy of the multivariate analysis-based model. Here we selected multiple marker combinations having AUC ≧ 0.80 as markers that influence the distinction between liver cancer groups and controls. Statistical analysis was performed using SPSS ver. 23 (IBM, USA) and R (Ver. 3.4.1).

### EXAMPLE 4: Biomarkers finally identified

### EXAMPLE 4-1: Identification of markers for early diagnosis of liver cancer by applying real individual samples.

One hundred seventy-seven proteins as the target marker and 385 peptides therefrom as described in Example 2-3 were tested on each sample from Asan medical center by MRM analyses. From the pretreatment of the clinical samples for MRM analysis to the orders of injecting the sample to mass spectrometers, an experimental design by blinding and randomization batch was established. As a first batch of the experiment, MRM analysis were done on each of the blood samples from a total of 150 subjects (50 hepatitis B, 50 cirrhosis, 50 liver cancer patients). As a second batch of the experiment, MRM analysis were done on each of the blood samples from a total of 150 subjects (50 hepatitis B, 50 cirrhosis, 50 liver cancer patients). As a third batch of the experiment, MRM analysis were done on each of the blood samples from a total of 140 subjects (57 hepatitis B, 43 cirrhosis, 40 liver cancer patients). MRM analyses were performed basically in the same way as described in EXAMPLE 3.

Briefly, all the analyses were done using 6490 triple quadrupole (QqQ) mass spectrometer (Agilent Technologies, USA) equipped with Jetstream electrospray and coupled to 1260 Capillary LC system (Agilent) consisting of two pumps. The data from MRM were analyzed by Skyline.

The final markers were identified through univariable analysis and multivariable analysis for 177 proteins selected in Example 2 and 385 peptides derived therefrom. In univariate analysis, liver cancer group was compared with high risk group (HBV + LC), hepatitis B group (HBV), and liver cirrhosis group (LC) to evaluate and confirm the differences in expression patterns and individual diagnostic powers for single markers.

The final markers were determined through univariate and multivariate analysis of the above identified marker candidate group. The univariate and multivariate analyzes are described in Examples 4-2 and 4-3, respectively, whereby the final identified markers are described in Tables 1-1, 1-2 and 1-3 described above, in which AUC values are expressed as mean values.

### EXAMPLE 4-2: Identification of markers through univariable analysis

The 177 proteins and 385 peptides selected in Example 2-3 were applied to individual samples to derive markers that can be used as single markers.

The liver cancer group was compared 1: 1 to each of high-risk group (HBV + LC), the hepatitis B group (HBV), and the cirrhosis group (LC), and the changes in the expression pattern of each individual marker compared to the control were determined
Hepatic cancer surveillance diagnostic marker candidates of 177 proteins and 385 peptides were analyzed by logistic regression 1:1 in three comparative groups (hepatitis B vs. liver cancer, cirrhosis vs. liver cancer, hepatitis B + liver cirrhosis vs. liver cancer). By using AUC values by ROC curves, single markers that differ between comparison groups were identified. Statistical analysis was performed using SPSS ver. 23 (IBM, USA) and R (Ver. 3.4.1). Marker candidates for surveillance / diagnosing liver cancer were selected only for the markers that showed a difference in the expression in the comparison and have the same expression pattern with AUC ≧ 0.60 in the first, second, and third experiments in the three comparison groups (Refer to Tables 3-1 and 3-2). As a result, a total of 55 proteins and 122 peptides therefrom that showed a differential expression in the high-risk group of developing liver cancer in comparison to liver cancer group were identified/selected as listed in in Table 3-1 and 3-2.

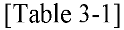

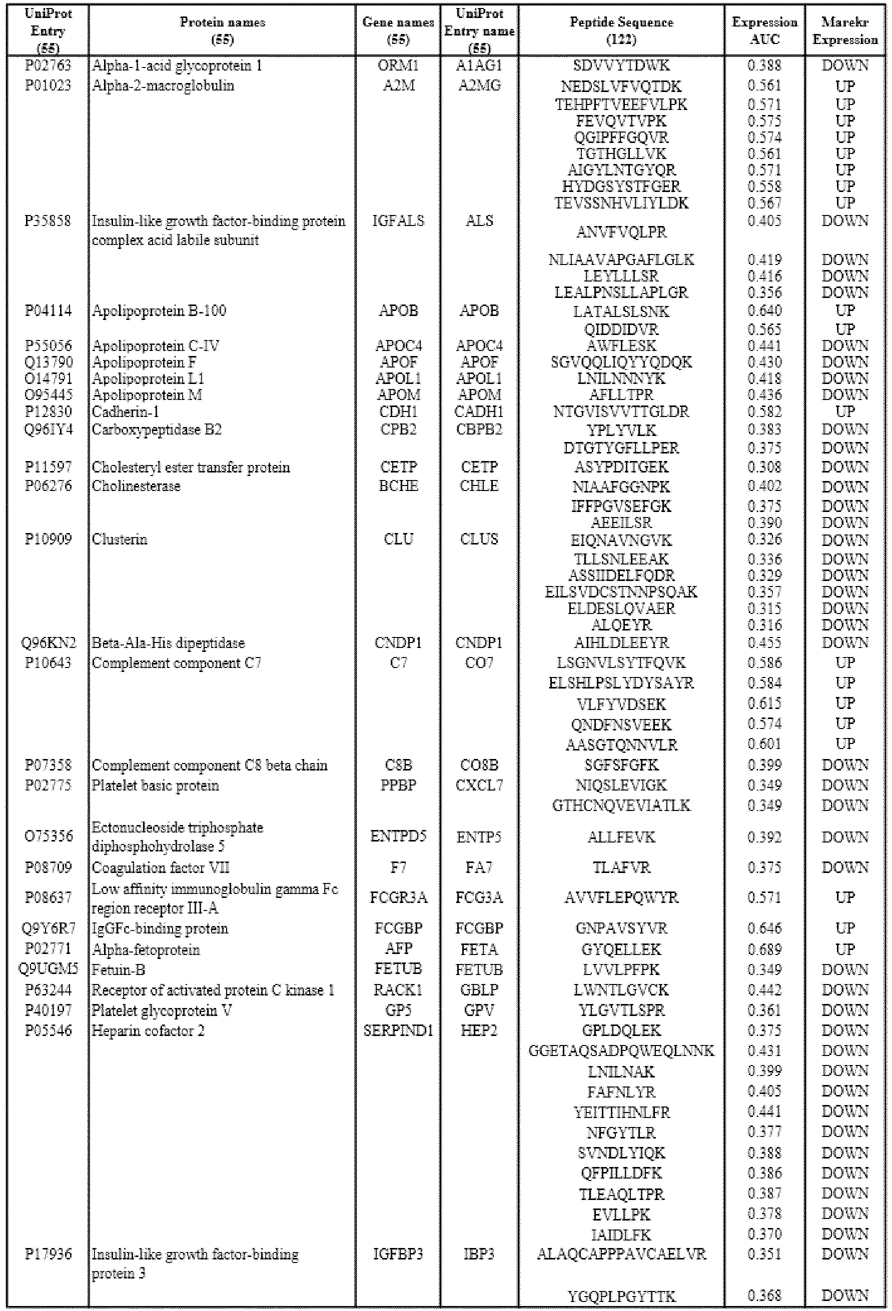

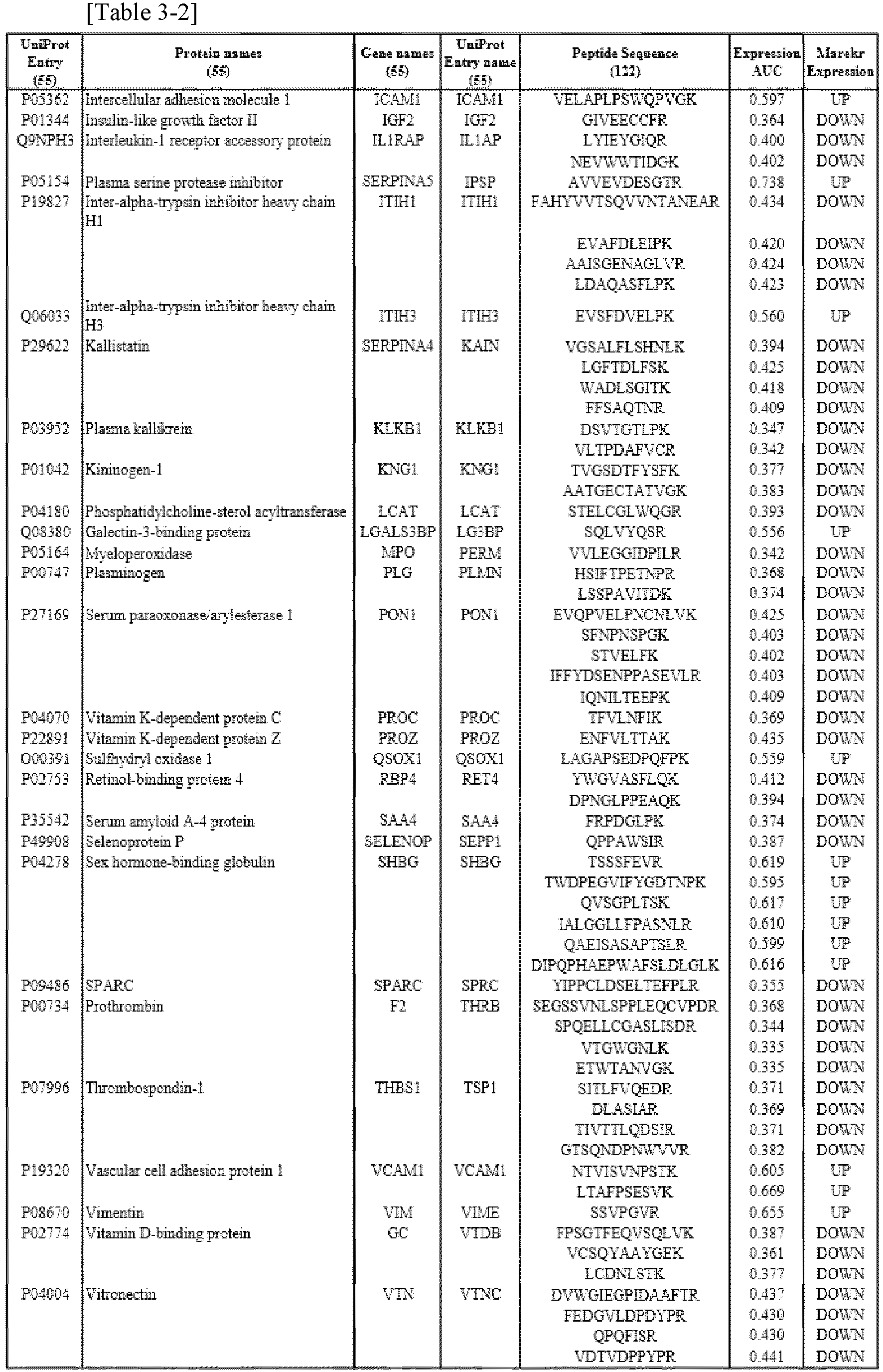

Among the 55 markers as described above, particularly 118 peptides from 52 markers were found to be differentially expressed in the comparison group of Hepatitis B vs HCC, which are listed in Table 4-1 and 4-2. Among the 52 markers and 118 peptides, 30 peptides from 13 proteins showed higher expression in liver cancer group, and 88 peptides from 39 proteins showed higher expression in Hepatitis B group. In particular, 6 proteins VCAM1, C7, IGFALS, CLU, PPBP7, and SERPIND1 were confirmed to have AUC≥ 0.70 in all three experiments. Among these, VCAM1 and C7 were highly expressed in liver cancer group and IGFALS, CLU, PPBP7, and SERPIND1 were highly expressed in hepatitis B group.

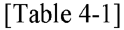

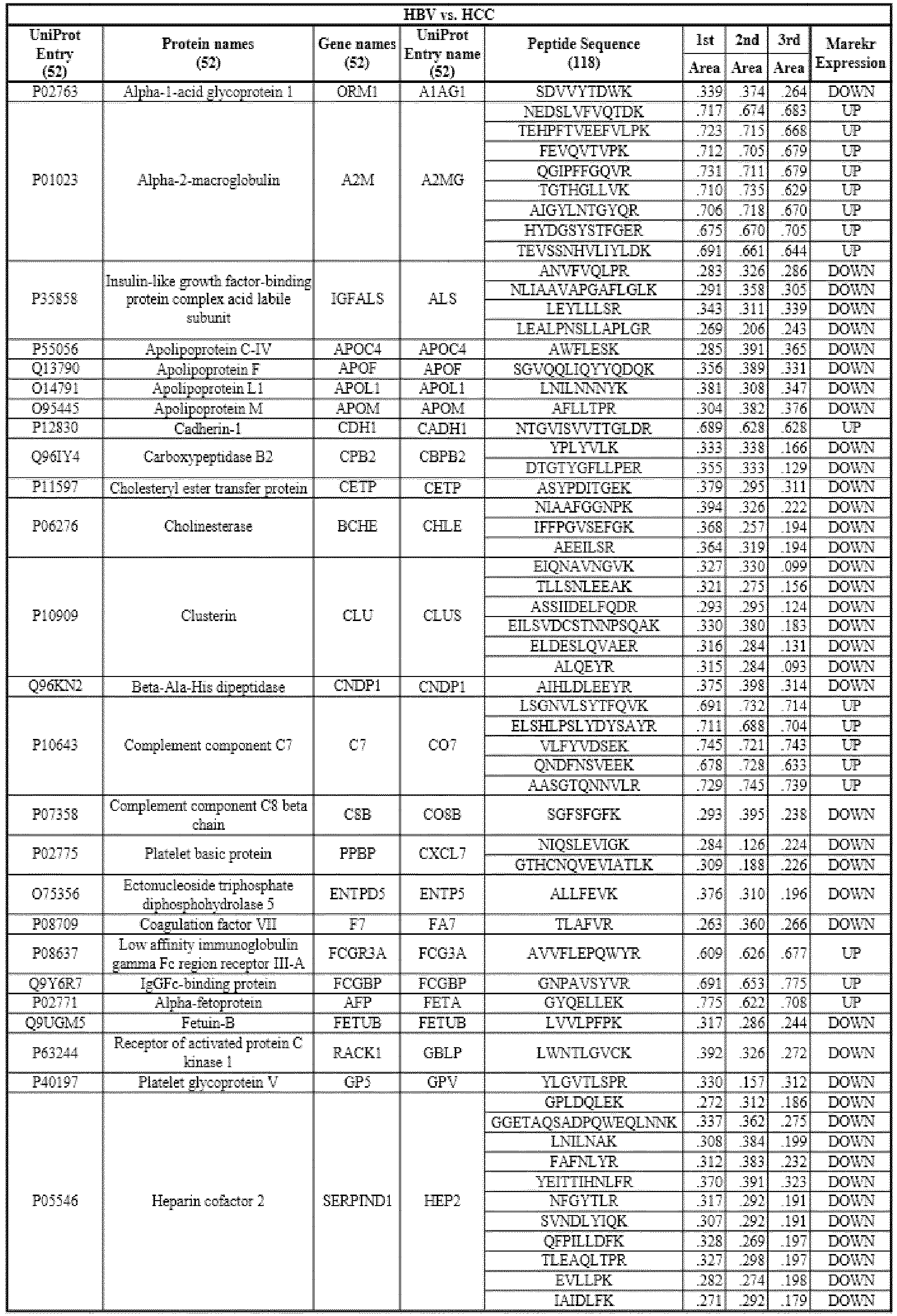

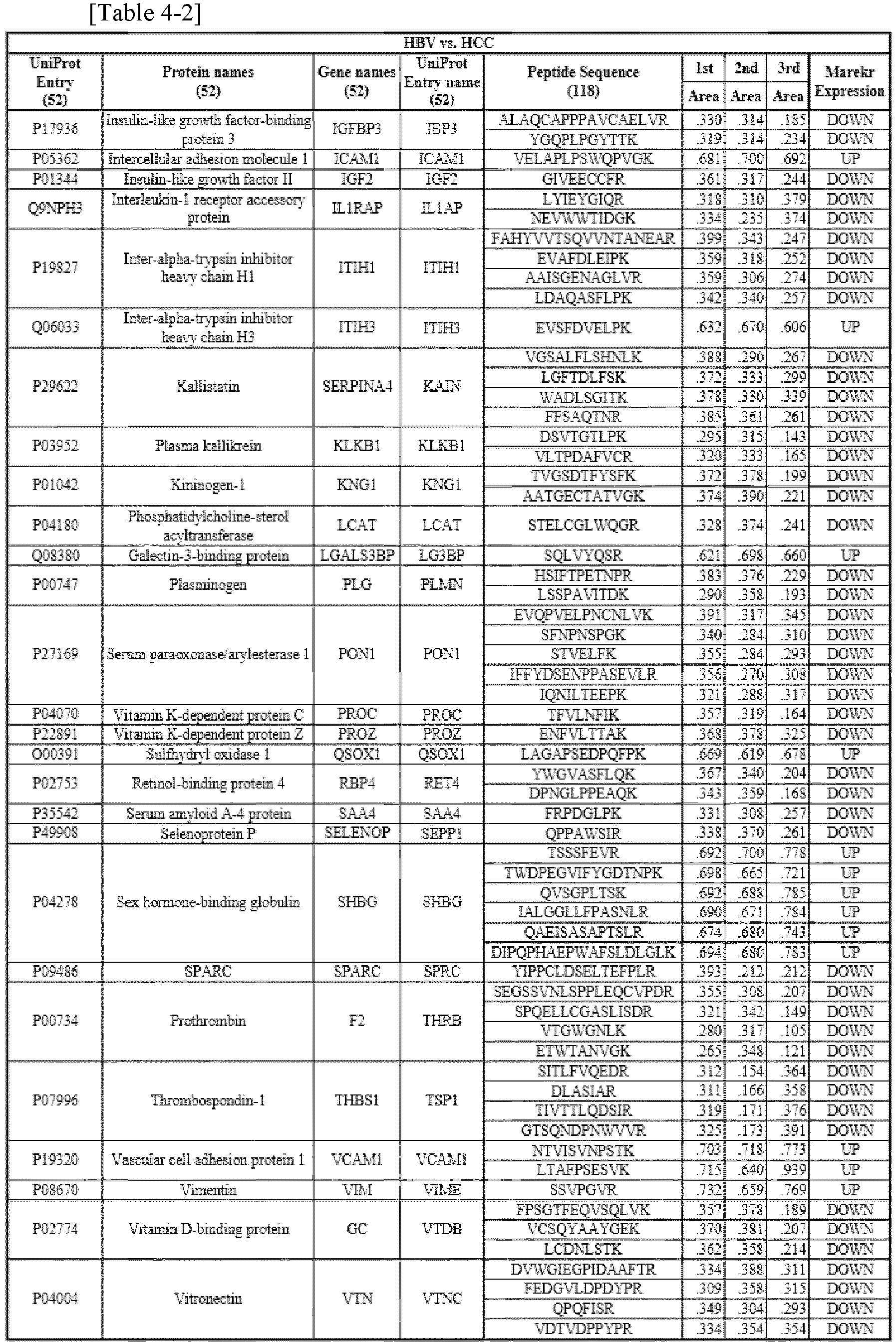

Among the 55 markers, in particular, the cirrhosis group (LC) vs. HCC comparison identified six peptides from the five proteins APOB, AFP, SERPINA5, CETP, and MPO to have AUC ≧ 0.60 in three experiments and are shown in Table 4-3. Markers (APOB, AFP, SERPINA5) was highly expressed in the liver cancer group, two markers (CETP, MPO) was confirmed to be highly expressed in the cirrhosis group.

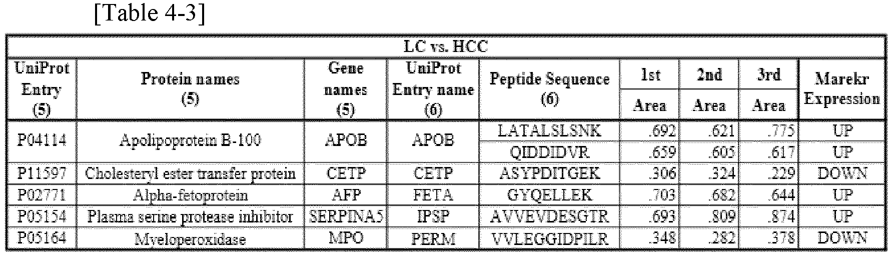

Among the 55 markers, in particular, liver cancer high risk group (HBV + LC) vs. liver cancer group (HCC) comparison identified four peptides of four proteins, AFP, SERPINA5, CETP and FETUB to have AUC ≥ 0.60 in three experiments and are shown in Table 4-4. AFP and SERPINA5 were highly expressed in liver cancer group, and CETP and FETUB were highly expressed in liver cancer high risk group. AFP was highly expressed in liver cancer group with AUC ≥ 0.70 in the first experiment, and CETP, FETUB, and SERPINA5 were identified in the third experiment. SERPINA5 showed very high discrimination ability with AUC = 0.915 in the third experiment.

Liver cancer high risk group (hepatitis group + cirrhosis group) were analyzed by integrating the data of the two disease groups. Clinically, hepatitis B is also a cause of liver cirrhosis, so the samples used in the study may also include a sample of a patient who has progressed to liver cirrhosis through hepatitis B. However, there are cases where hepatitis B progresses directly to liver cancer. Therefore, even if hepatitis progresses to cirrhosis, the cirrhosis does not include hepatitis. This is because cirrhosis does not represent the condition of hepatitis in the past, but only represent the current cirrhosis.

The markers described above can be advantageously used for early diagnosis of liver cancer of the subject with hepatis or the subject with chronic hepatitis not diagnosed with cirrhosis.

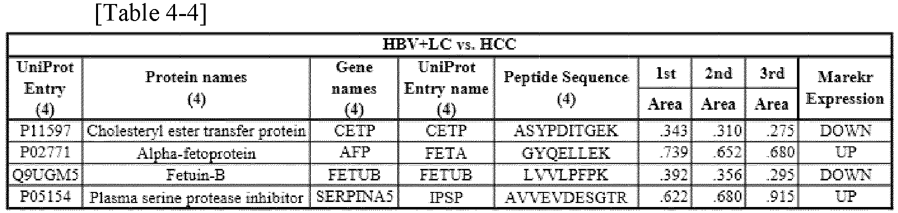

### EXAMPLE 4-3. Identification of markers through multivariable analysis

For the three comparison group (Hepatitis B vs. Liver cancer, Cirrhosis vs. Liver cancer, Hepatitis B + Cirrhosis vs. Liver cancer), multivariate analysis was performed at the same time for the 385 peptides from 177 proteins makers of liver cancer surveillance to confirm the cause and effect relationship between multiple markers and the combination of multiple markers having a cause and effect relationship with single markers,

For multivariate analysis, data that combines the first and second experiments were used to prevent the confounding effect of various variables. The third experimental data was used as independent data to verify the multivariate analysis-based model derived from the first and second experimental results. The model of combination of the markers influential in the development of liver cancer by multivariate analysis can distinguish between the liver cancer group and the control group and depending on the analysis method, the markers affecting the results may be selected and combined differently. To compensate for this, two kinds of methods, i.e., logistic regression and support vector machines were used. Models selected and combined by multivariate analysis can distinguish between liver cancer group and control group with higher diagnostic power and accuracy than single markers. However, if a model is made using all of the given data, there may be a possibility that the model will produce good results in the current data set but bad results in the new data set for reasons such as overfitting. To solve this problem, the resampling method was used. In reference to Kim H. et al., Sci Rep. 2017 Aug 25;7(1):9449 and Kim H. et al., Mol Cell Proteomics. 2017 Jul;16(7): 1312-1323, the present model by multivariate analysis was validated using the cross-validation method in 5-fold and 10-fold. In reference to Meyers R Let. al., Lancet Oncol. 2017 Jan; 18(1): 122-131, the present model by multivariate analysis was validated by bootstrap method, which splits the data at ratios of 0.7 and 0.9. Finally, ROC curve and accuracy were used to confirm the diagnostic power and accuracy of the multivariate analysis-based model. Statistical analysis was performed using SPSS ver. 23 (IBM, USA) and R (Ver. 3.4.1).

As a result of the multivariate analysis, it was confirmed that the present model of combination of multiple markers with cause and effect relationship including single markers showing differential expression in comparison to liver cancer group can distinguish between the liver cancer group and the control group with improved diagnostic power compared to the single markers.

In one embodiment of the present disclosure, marker candidates for liver cancer surveillance / diagnosis are the markers that can be combined with AUC ≧ 0.80 determined by two analytical methods and resampling method (refer to Table 5-1 and 5-2). As a result, a total of 81 proteins and 121 peptides derived therefrom were selected, which can be combined into a model for distinguishing a liver cancer group from a high-risk group, and are shown in Tables 5-1 and 5-2.

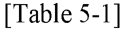

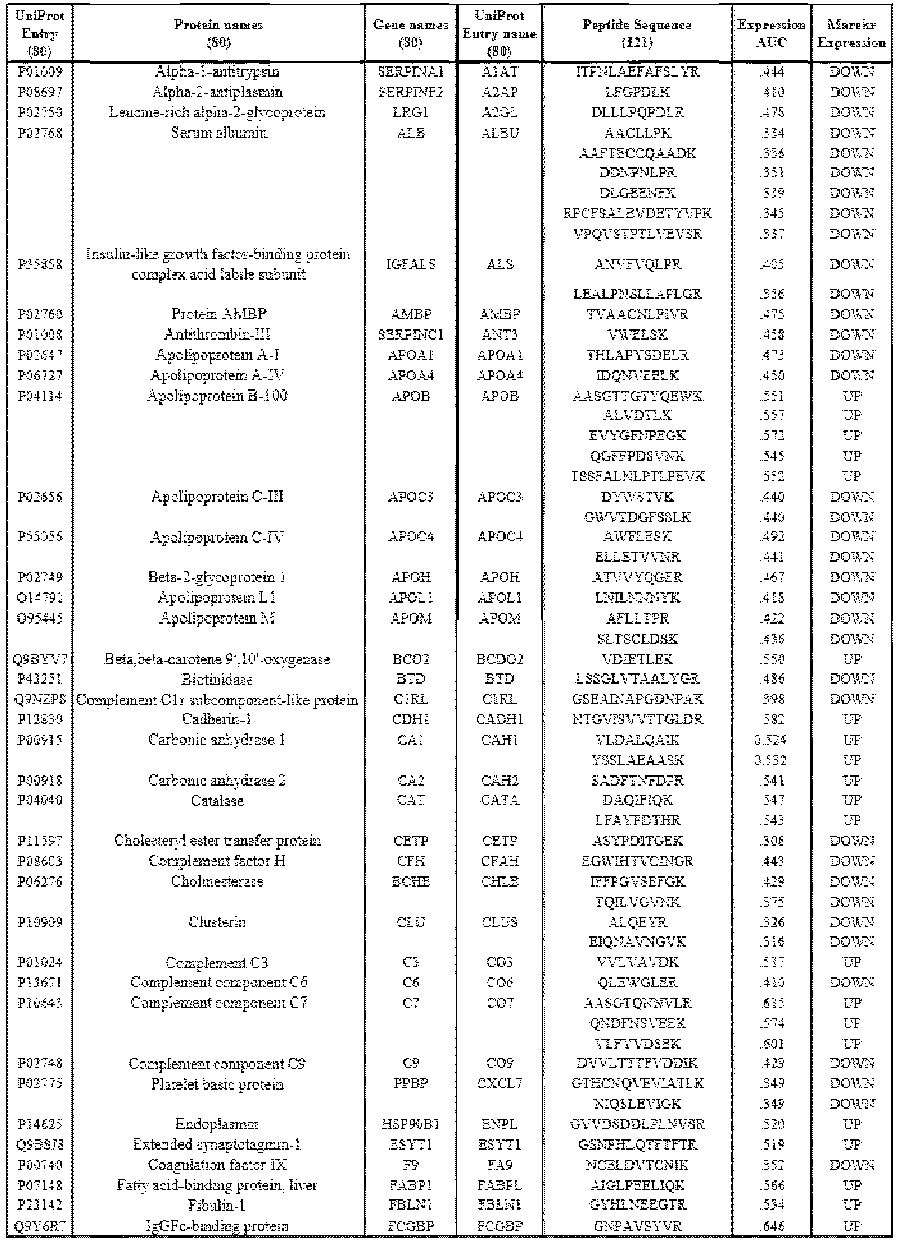

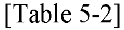

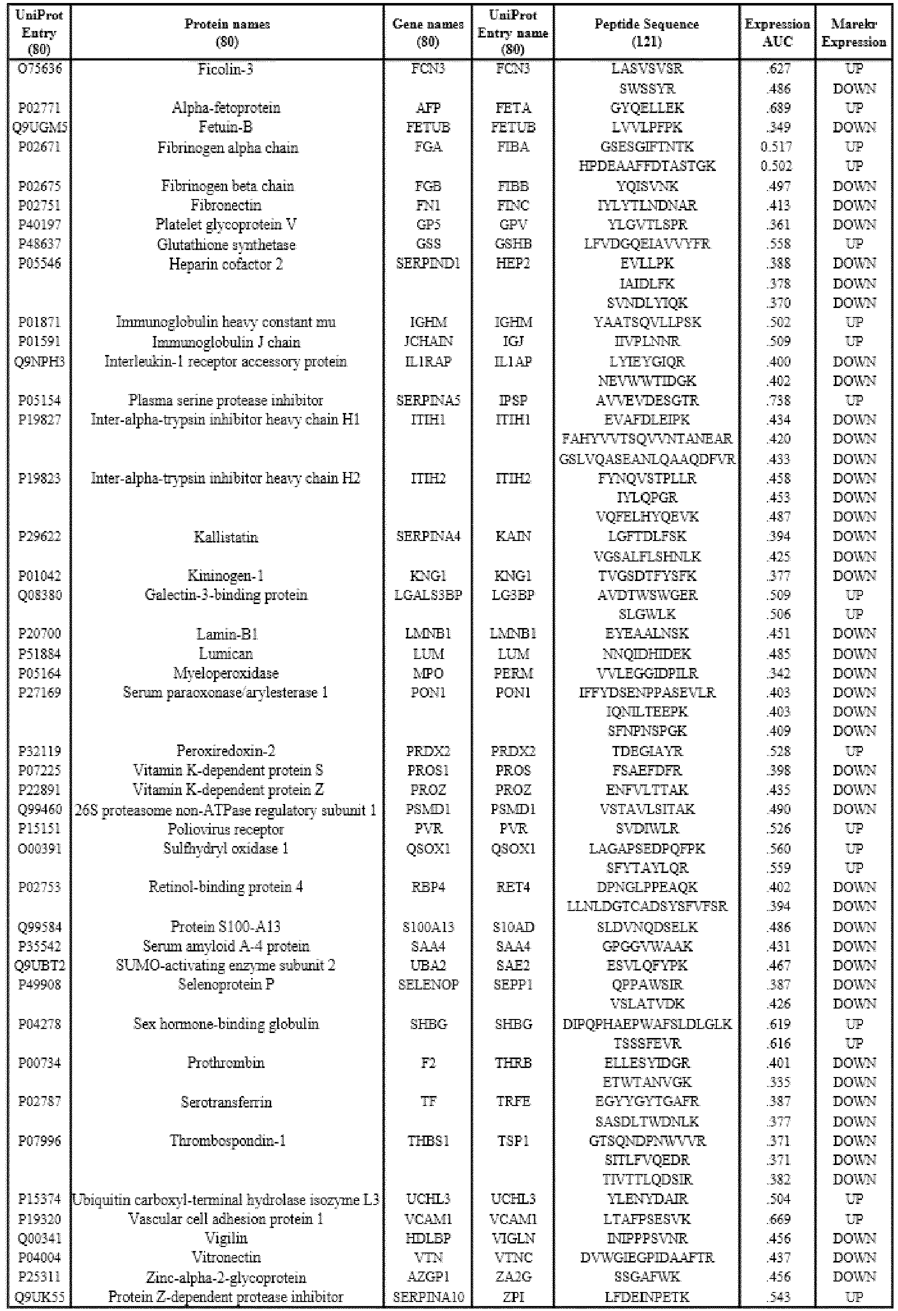

### EXAMPLE 4-4. Generation of Multiple Marker Combinations Using Selected Markers in Multivariate Analysis

From the list of markers selected in Example 4-3 (see Tables 5-1 and 5-2), the models of combination of multiple markers were developed based on the AUC and Accuracy values determined by logistic regression and support vector machine for "Liver cancer high risk group (hepatitis + cirrhosis) vs. Liver cancer group, Cirrhosis group vs. Liver cancer group, Hepatitis B group vs. Liver cancer group".

In two resampling methods and two analytical methods for the three comparison groups, 24 models of combination of marker influential in the development of liver cancer with AUC ≥ 0.80 were developed.

Among these, when cross-validation was used, 12 models in which multiple markers were combined and identified as AUC ≥ 0.80 are shown in Table 6-1.

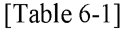

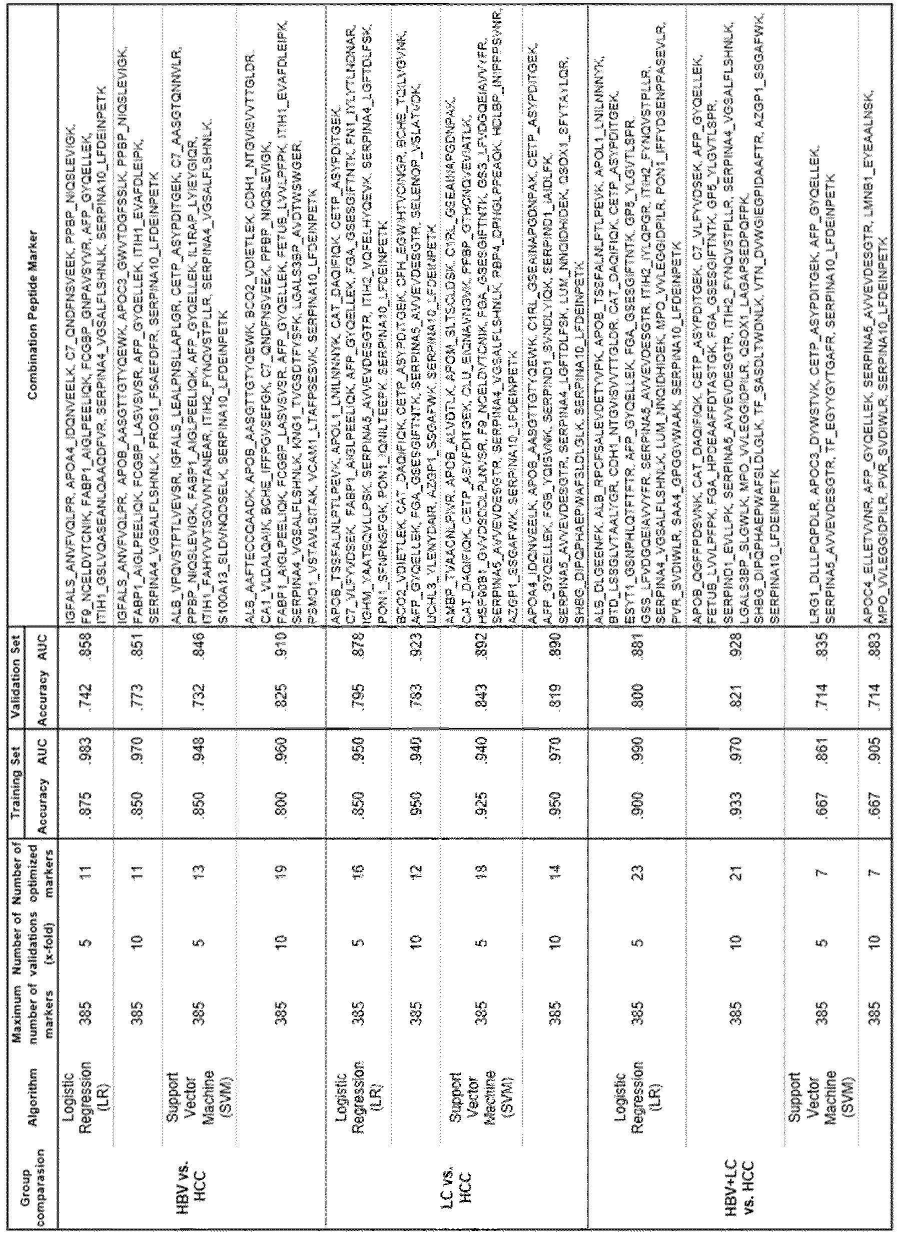

When bootstrap was used, 12 models in which multiple markers were combined and identified with AUC≥ 0.80 are shown in Table 6-2.

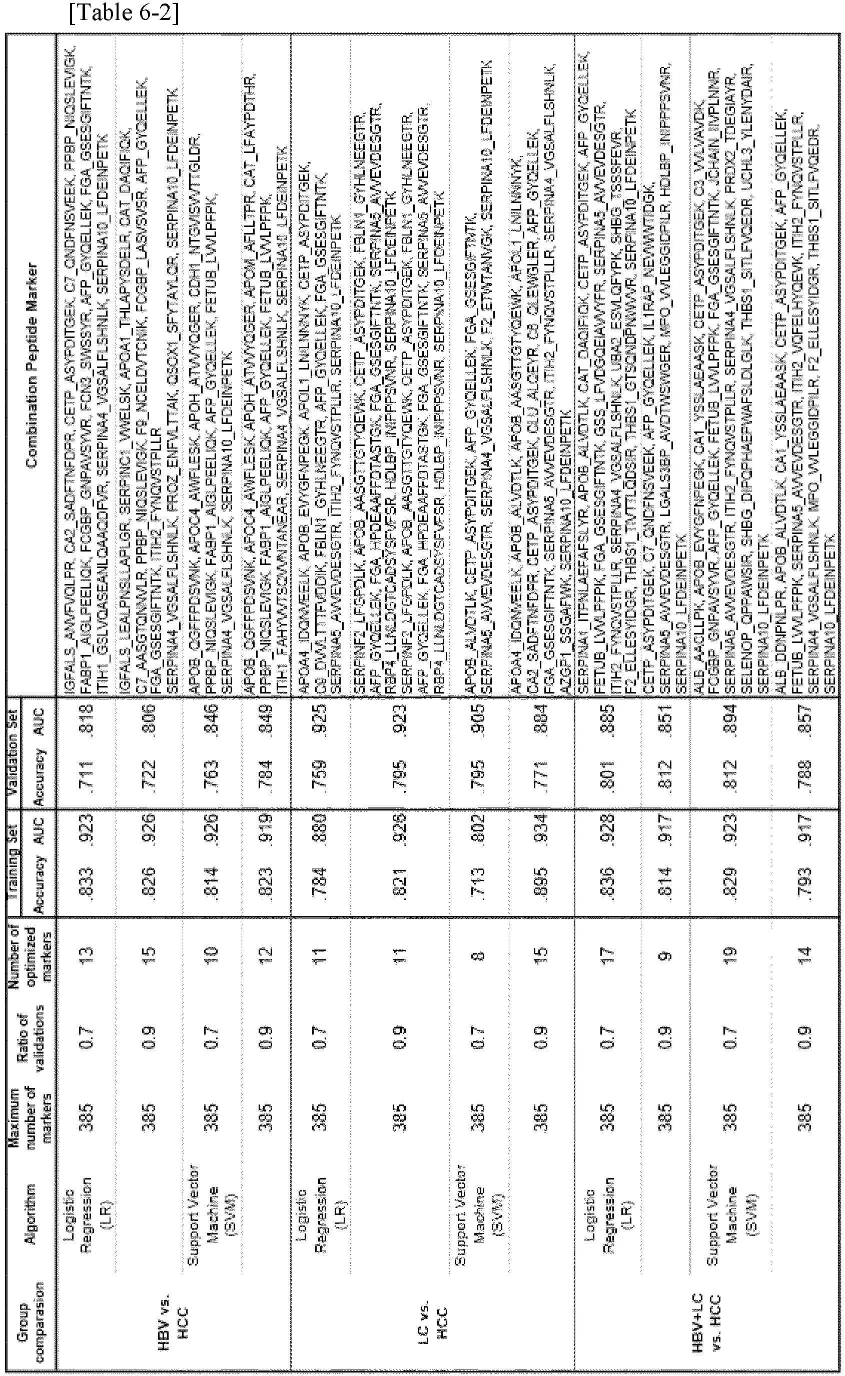

In Tables 6-1 and 6-2, the model showing the highest AUC and Accuracy among the models in which multiple markers are combined by two analysis methods is the model of combination of 21 markers with AUC = 0.970 and Accuracy = 0.933 by logistic regression method, and the model of combination of 19 markers with AUC=0.923, and Accuracy=0.874 by support vector machine method. In the third set (Validation set), the 21-marker combination model by logistic regression was identified as AUC = 0.928 and Accuracy = 0.821. The 19 marker combination models by the support vector machine was validated with AUC = 0.894 and Accuracy = 0.812. The markers, CETP, FGA, SERPINA5, MPO, SERPINA10, SHBG, ALB, APOB, AFP, ITIH2, SERPINA4, PVR, FETUB, TF, F2, THBS1 are commonly combined in two analytical methods (logistic regression and support vector machine).

In the combination of the markers disclosed in Table 6-1 and Table 6-2, when the high-risk liver cancer group and the liver cancer group (HBV + LC vs. HCC) were compared, 47 proteins and 62 peptides were determined as combinable markers. The list of markers used for the combination is shown in Table 7-1.

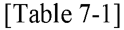

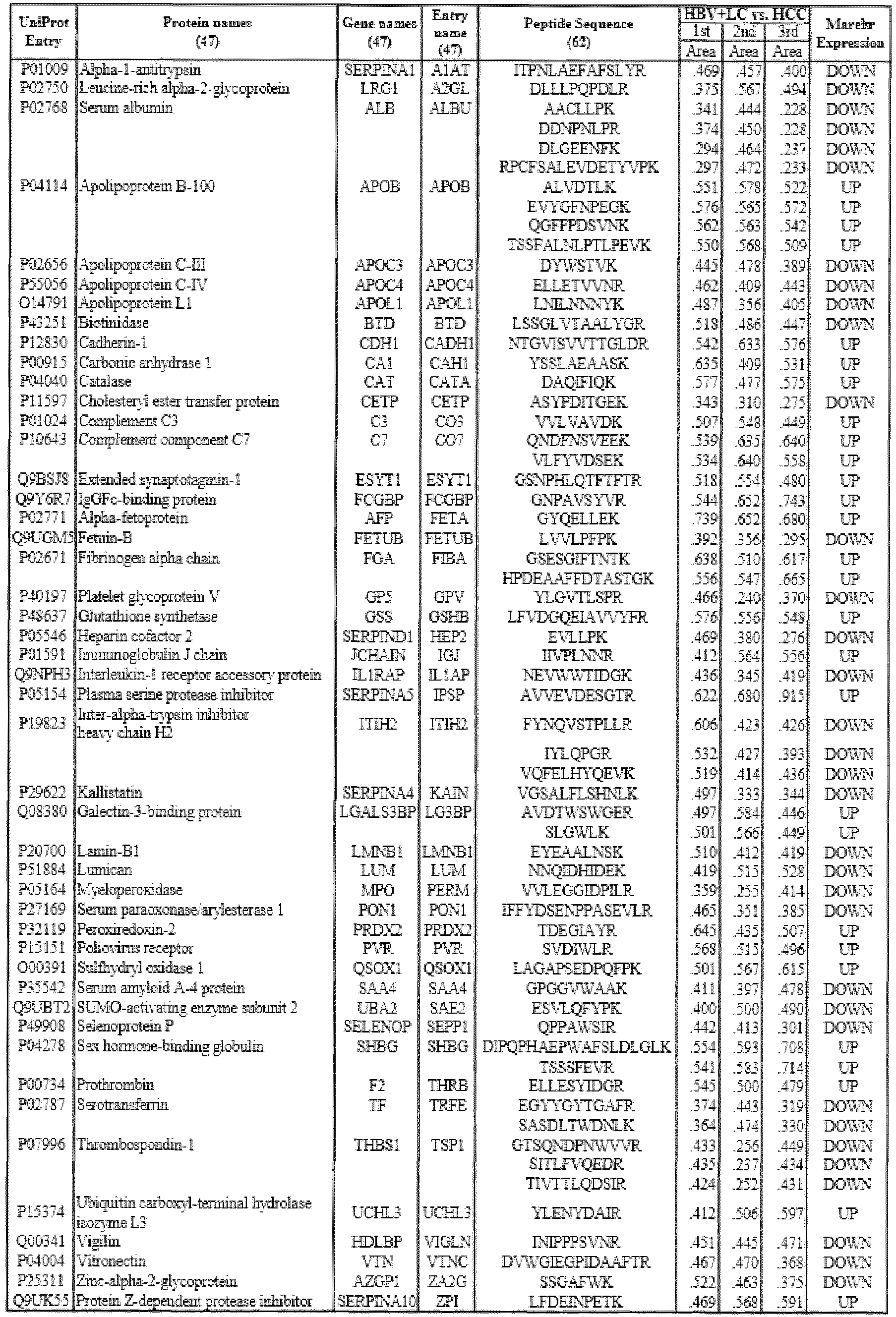

In the combination of the markers disclosed in Table 6-1 and 6-2, when the cirrhosis group (LC) and liver cancer group (HCC) (LC vs. HCC) were compared, 43 proteins and 53 peptides were determined as combinable markers. The list of markers used for the combination is shown in Table 7-2.

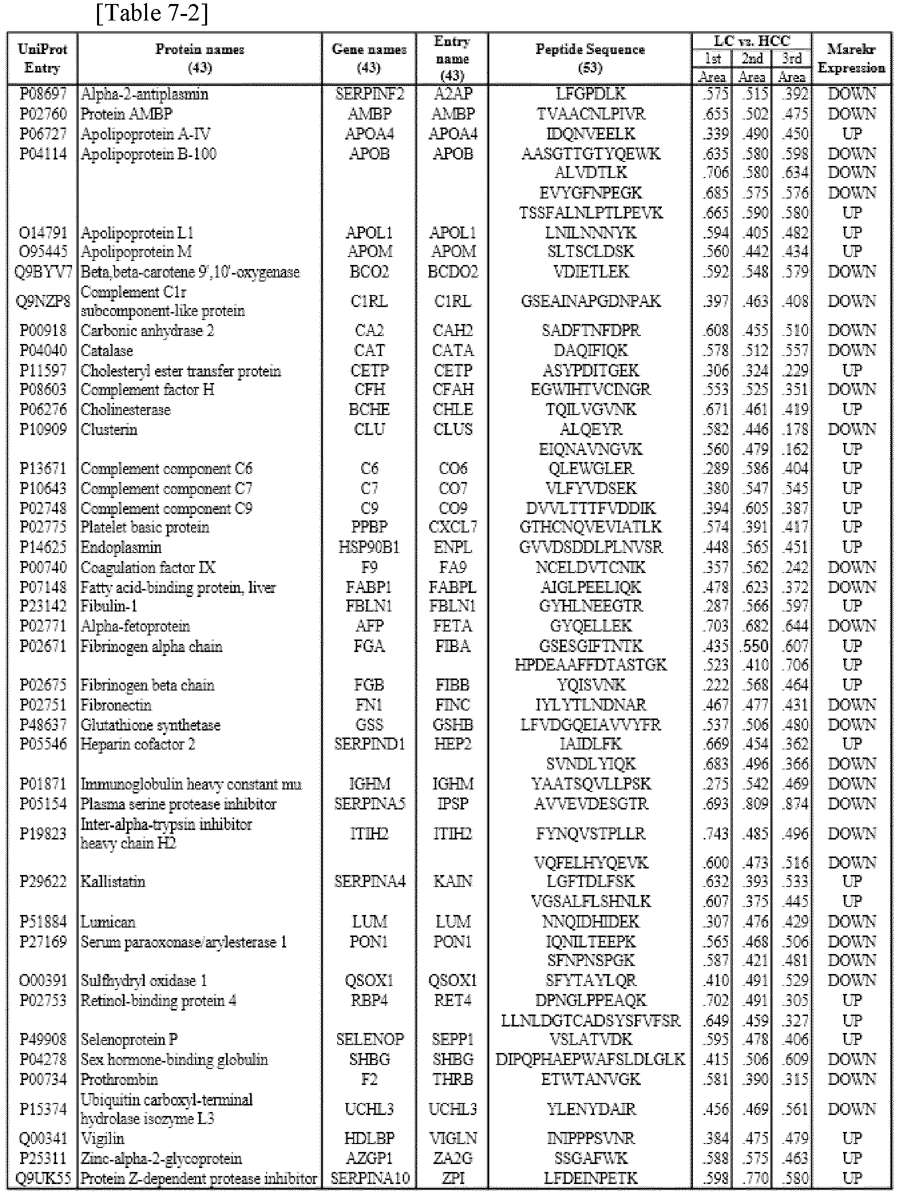

When 12 or more markers were combined among the markers listed in Table 6-1 and Table 6-2, it showed an excellent diagnostic power of AUC> 0.90, and thus the third data (Validation set) was confirmed. CAT, APOL1, RBP4, HDLBP, AZGP1, CETP, FGA, APOB, SERPINA5, SERPINA4, SERPINA10, APOA4, AFP, and ITIH2 were commonly combined in logistic regression and support vector machine methods

The model with the highest AUC and Accuracy among the models with multiple markers by the two analysis methods was the model with AUC = 0.940 and Accuracy = 0.950 by the logistic regression in which 12 markers were combined and the model of 14 marker combination with AUC = 0.970 and Accuracy = 0.950 by support vector machine. In the third set (Validation set), the AUC values were 0.923 and 0.890 and the Accuracy values were 0.783 and 0.819 for 12 marker combination models of logistic regression and 14 marker combination models by a support vector machine, respectively, verifying the diagnostic power and accuracy of the present models.

In the combination of the markers disclosed in Table 6-1 and 6-2, when the Hepatitis group (HBV) and liver cancer group (HCC) (HBV vs. HCC) were compared, 39 proteins and 47 peptides were determined as combinable markers. The list of markers used for the combination is shown in Table 7-3.

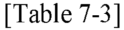

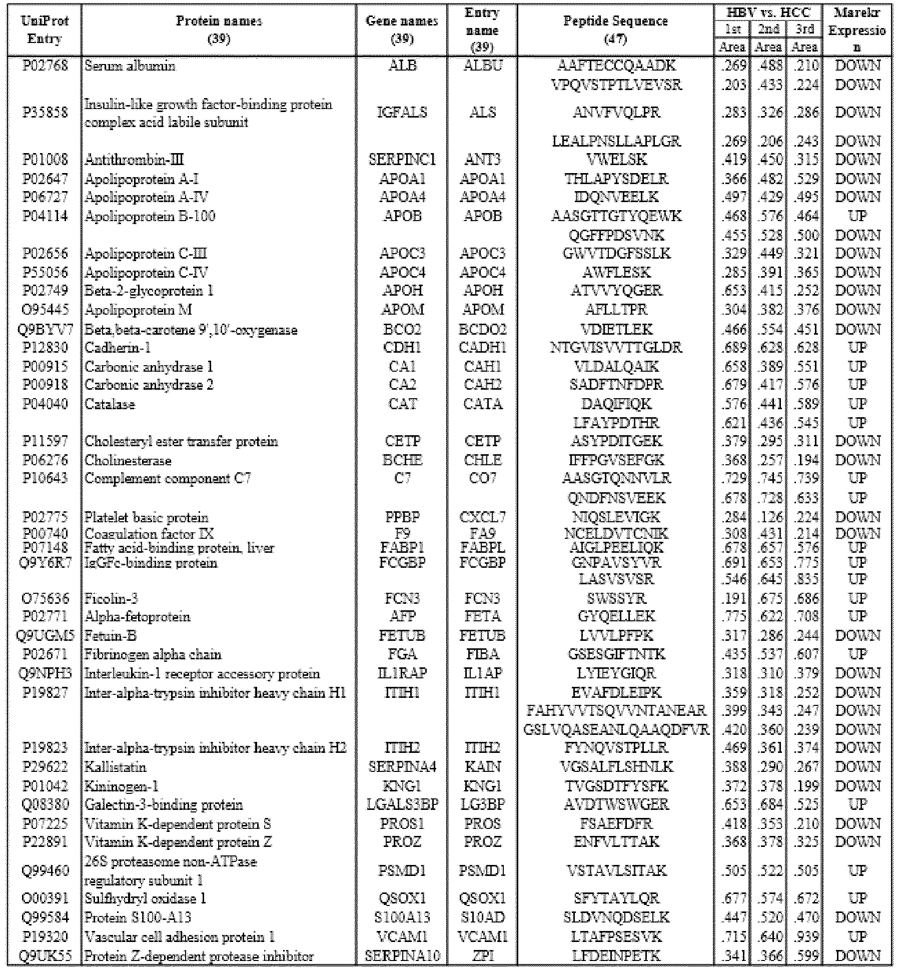

In Tables 6-1 and 6-2, the markers, APOB, CETP, AFP, ITIH2, SERPINA4, SERPINA10, CAT, IGFALS, C7, PPBP, FABP1, FCGBP, and ITIH1 were combined in common by two algorithms (logistic regression and support vector machine). Among the models with multiple markers combined by the two analysis methods, the model with the highest AUC and Accuracy was a model with AUC = 0.970 and Accuracy = 0.930 with 11 markers by logistic regression, and a model with AUC = 0.960 and Accuracy = 0.800 with 19 markers by support vector machine. When the two models were validated on the third set (Validation set), the 11-marker combination model was validated with AUC and Accuracy of 0.851 and 0.773, respectively, and the 19-marker combination model showed AUC and Accuracy of 0.910 and 0.825, respectively, which proved their diagnostic power and accuracy.

In the multiple marker combinations listed in Tables 6-1 and 6-2 above, the combination list of markers in the multivariate based models whose diagnostic power is AUC ≥ 0.90, accuracy ≥ 0.80 and which was verified with AUC ≥ 0.85 in the validation set is shown in Table 8.

As a result of multivariate analysis, it was confirmed that the combination of multiple markers rather than individual single markers can improve the diagnostic power in the high-risk group of liver cancer compared to the liver cancer group.

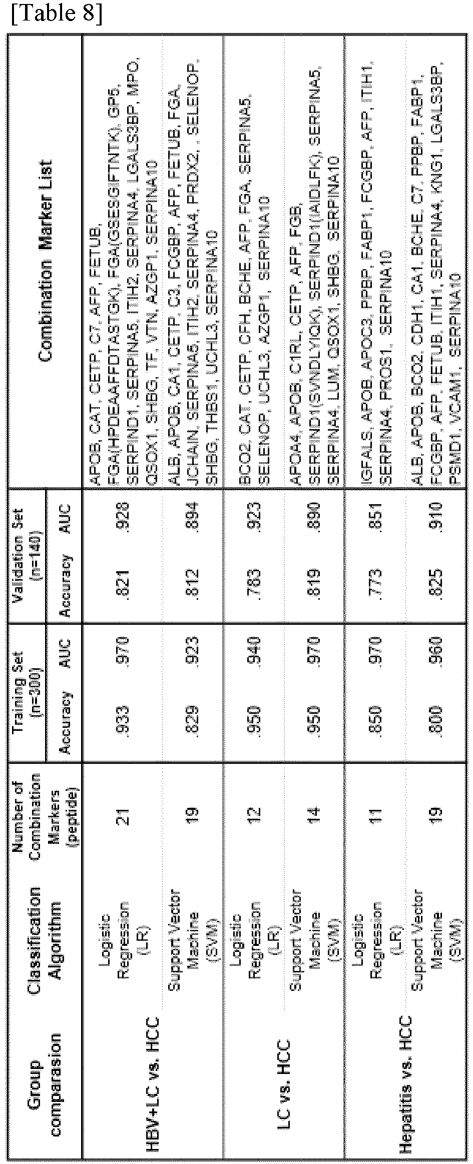

In the present invention, using a total of 440 serum samples, 100 proteins and 196 peptides therefrom showing differential expression patterns in comparison of high risk of liver cancer group: hepatitis B (HBV) group and liver cirrhosis (LC) group with liver cancer (HCC) group were identified. Such markers or combinations of the markers can be advantageously used as markers for surveillance and early diagnosis of liver cancer in high-risk liver cancer patients.

The various singular/plural permutations may be expressly set forth herein for sake of clarity. Although a few embodiments of the present disclosure have been shown and described, it would be appreciated by those skilled in the art that changes may be made in this embodiment without departing from the principles and sprit of the invention, the scope of which is defined in the claims and their equivalents.

Unless defined or interpreted otherwise, all technical and scientific terms and any acronyms used herein have the same meanings as commonly understood by one of ordinary skill in the art in the field of the invention. The contents of all publications disclosed as references herein are incorporated herein by reference.

## Claims

1. A biomarker for monitoring or early diagnosis of liver cancer of a subject at high risk of developing liver cancer in a blood sample, the biomarker is at least one biomarker selected from the group consisting of: ORM1 (Alpha-1-acid glycoprotein 1), SERPINA1 (Alpha-1-antitrypsin), SERPINF2 (Alpha-2-antiplasmin), A2M (Alpha-2-macroglobulin), ALB (Serum albumin), SERPINC1 (Antithrombin-III), APOA1 (Apolipoprotein A-I), APOA4 (Apolipoprotein A-IV), APOB (Apolipoprotein B-100), APOC3 (Apolipoprotein C-III), APOC4 (Apolipoprotein C-IV), APOF (Apolipoprotein F), APOH (Beta-2-glycoprotein 1), APOL1 (Apolipoprotein L1), APOM (Apolipoprotein M), BCO2 (Beta,beta-carotene 9',10'-oxygenase), BTD (Biotinidase), C1RL (Complement C1r subcomponent-like protein), CDH1 (Cadherin-1), CA1 (Carbonic anhydrase 1), CA2 (Carbonic anhydrase 2), CAT (Catalase), CPB2 (Carboxypeptidase B2), CETP (Cholesteryl ester transfer protein), CFH (Complement factor H), C3 (Complement C3), C8B (Complement component C8 beta chain), C9 (Complement component C9), PPBP (Platelet basic protein), HSP90B1 (Endoplasmin), ENTPD5 (Ectonucleoside triphosphate diphosphohydrolase 5), ESYT1 (Extended synaptotagmin-1), F7 (Coagulation factor VII), F9 (Coagulation factor IX), FABP1 (Fatty acid-binding protein, liver), FCGBP (IgGFc-binding protein), FETUB (Fetuin-B), FGA (Fibrinogen alpha chain), FGB (Fibrinogen beta chain), RACK1 (Receptor of activated protein C kinase 1), GP5 (Platelet glycoprotein V), GSS (Glutathione synthetase), SERPIND1 (Heparin cofactor 2), ICAM1 (Intercellular adhesion molecule 1), IGHM (Immunoglobulin heavy constant mu), IL1RAP (Interleukin-1 receptor accessory protein), ITIH2 (Inter-alpha-trypsin inhibitor heavy chain H2), ITIH3 (Inter-alpha-trypsin inhibitor heavy chain H3), KNG1 (Kininogen-1), LMNB1 (Lamin-B1), MPO (Myeloperoxidase), PON1 (Serum paraoxonase/arylesterase 1), PRDX2 (Peroxiredoxin-2), PROC (Vitamin K-dependent protein C), PROS1 (Vitamin K-dependent protein S), PROZ (Vitamin K-dependent protein Z), PSMD1 (26S proteasome non-ATPase regulatory subunit 1), PVR (Poliovirus receptor), RBP4 (Retinol-binding protein 4), S100A13 (Protein S100-A13), SAA4 (Serum amyloid A-4 protein), UBA2 (SUMO-activating enzyme subunit 2), SELENOP (Selenoprotein P), SPARC (SPARC), TF (Serotransferrin), THBS1 (Thrombospondin-1), UCHL3 (Ubiquitin carboxyl-terminal hydrolase isozyme L3), VCAM1 (Vascular cell adhesion protein 1), HDLBP (Vigilin), VIM (Vimentin), AZGP1 (Zinc-alpha-2-glycoprotein) and SERPINA10 (Protein Z-dependent protease inhibitor).

2. The biomarker of claim 1, wherein the biomarker further comprises at least one biomarker selected from the group consisting of: LRG1 (Leucine-rich alpha-2-glycoprotein), IGFALS (Insulin-like growth factor-binding protein complex acid labile subunit), AMBP (Protein AMBP [Cleaved into: Alpha-1-microglobulin), BCHE (Cholinesterase), CLU (Clusterin), CNDP1 (Beta-Ala-His dipeptidase), C6 (Complement component C6), C7 (Complement component C7), FBLN1 (Fibulin-1), FCGR3A (Low affinity immunoglobulin gamma Fc region receptor III-A), FCN3 (Ficolin-3), AFP (Alpha-fetoprotein), FN1 (Fibronectin), IGFBP3 (Insulin-like growth factor-binding protein 3), IGF2 (Insulin-like growth factor II), JCHAIN (Immunoglobulin J chain), SERPINA5 (Plasma serine protease inhibitor), ITIH1 (Inter-alpha-trypsin inhibitor heavy chain H1), SERPINA4 (Kallistatin), KLKB1 (Plasma kallikrein), LCAT (Phosphatidylcholine-sterol acyltransferase), LGALS3BP (Galectin-3-binding protein), LUM (Lumican), PLG (Plasminogen), QSOX1 (Sulfhydryl oxidase 1), SHBG (Sex hormone-binding globulin), F2 (Prothrombin), GC (Vitamin D-binding protein) and VTN (Vitronectin).

3. The biomarker of claims 1 or 2, wherein the subject at high risk of developing liver cancer includes a hepatitis patient or a liver cirrhosis patient.

4. The biomarker of any one of claims 1 to 3, wherein the biomarker is at least one biomarker selected from the group consisting of: SERPINA1, ALB, APOB, APOC3, APOC4, APOL1, BTD, CDH1, CA1, CAT, CETP, C3, ESYT1, FCGBP, FETUB, FGA, GP5, GSS, SERPIND1, IL1RAP, ITIH2, LMNB1 MPO, PON1, PRDX2, PVR, SAA4, UBA2, SELENOP, TF, THBS1, UCHL3, HDLBP, AZGP1 and SERPINA10.

5. The biomarker of claim 4, wherein the biomarker further comprises at least one biomarker elected from the group consisting of: LRG1, C7, AFP, JCHAIN, SERPINA5, SERPINA4, LGALS3BP, LUM, QSOX1, SHBG, F2, and VTN.

6. The biomarker of any one of claims 1 to 3, wherein the subject at high risk of developing liver cancer is a hepatitis patient, and the at least one biomarker for monitoring or early diagnosis of liver cancer for the hepatitis patient is selected from the group consisting of ORM1, A2M, ALB, SERPINC1, APOA1, APOA4, APOB, APOC3, APOC4, APOF, APOL1, APOM, APOH, BCO2, CDH1, CPB2, CA1, CA2, CAT, CETP, C8B, PPBP, F9, FABP1, ENTPD5, F7, FCGBP, FETUB, FGA, RACK1, GP5, SERPIND1, ICAM1, IGF2, IL1RAP, ITIH2, ITIH3, KNG1, PON1, PROC, PROS1, PROZ, PSMD1, RBP4, S100A13, SAA4, SELENOP, SPARC, THBS1, VCAM1, VIM and SERPINA10.

7. The biomarker of claim 6, wherein the biomarker further comprises at least one biomarker selected from the group consisting of IGFALS, BCHE, CLU, CNDP1, C7, FCGR3A, FCN3, AFP, IGFBP3, ITIH1, SERPINA4, KLKB1, LCAT, LGALS3BP, PLG, QSOX1, SHBG, F2, GC and VTN.

8. The biomarker of any one of claims 1 to 3, wherein the subject at high risk of developing liver cancer is a liver cirrhosis patient, and the at least one biomarker for monitoring or early diagnosis of liver cancer for the liver cirrhosis patient is selected from the group consisting of SERPINF2, APOA4, APOB, APOL1, APOM, BCO2, C1RL, CA2, CAT, CETP, CFH, C9, PPBP, HSP90B1, F9, FABP1, FGA, FGB, GSS, SERPIND1, IGHM, ITIH2, MPO, PON1, RBP4, SELENOP, UCHL3, HDLBP, AZGP1 and SERPINA10.

9. The biomarker of claim 8, wherein the biomarker further comprises at least one biomarker selected from the group consisting of AMBP, BCHE, CLU, C6, C7, FBLN1, AFP, FN1, SERPINA5, SERPINA4, LUM, QSOX1, SHBG, and F2.

10. The biomarker of any one of claims 1 to 3, wherein the at least one biomarker is a combination of the biomarkers selected from the group consisting of: ALB, APOB, APOL1, BTD, CDH1, CAT, CETP, ESYT1, AFP, FGA, GP5, GSS, SERPINA5, ITIH2, ITIH2, ERPINA4, LUM, MPO, PON1, PVR, SAA4, and SERPINA10;
APOB, CAT, CETP, C7, AFP, FETUB, FGA, GP5, SERPIND1, SERPINA5, ITIH2, SERPINA4, LGALS3BP, MPO, QSOX1, SHBG, TF, VTN, AZGP1, and SERPINA10;
LRG1, APOC3, CETP, AFP, SERPINA5, TF, and SERPINA10;
APOC4, AFP, SERPINA5, LMNB1, MPO, PVR, and SERPINA10;
SERPINA1, APOB, CAT, CETP, AFP, FETUB, FGA, GSS, SERPINA5, ITIH2, SERPINA4, UBA2, SHBG, F2, THBS1, THBS1, and SERPINA10;
CETP, C7, AFP, IL1RAP, SERPINA5, LGALS3BP, MPO, HDLBP, and SERPINA10;
ALB, APOB, CA1, CETP, C3, FCGBP, AFP, FETUB, FGA, JCHAIN, SERPINA5, ITIH2, SERPINA4, PRDX2, SELENOP, SHBG, THBS1, UCHL3, and SERPINA10; and
ALB, APOB, CA1, CETP, AFP, FETUB, SERPINA5, ITIH2, SERPINA4, MPO, F2, THBS1, and SERPINA10.

11. The biomarker of any one of claims 1 to 3, wherein the subject at high risk of developing liver cancer is a hepatitis patient, and the at least one biomarker for monitoring or early diagnosis of liver cancer for the hepatitis patient is a combination of the biomarkers selected from the group consisting of:
IGFALS, APOA4, C7, PPBP, F9, FABP1, FCGBP, AFP, ITIH1, SERPINA4, and SERPINA10;
IGFALS, APOB, APOC3, PPBP, FABP1, FCGBP, AFP, ITIH1, SERPINA4, PROS1, and SERPINA10;
ALB, IGFALS, CETP, C7, PPBP, FABP1, AFP, IL1RAP, ITIH1, ITIH2, SERPINA4, S100A13, and SERPINA10;
ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10;
IGFALS, CA2, CETP, C7, PPBP, FABP1, FCGBP, FCN3, AFP, FGA, ITIH1, SERPINA4, and SERPINA10;
IGFALS, SERPINC1, APOA1, CAT, C7, PPBP, F9, FCGBP, AFP, FGA, ITIH2, SERPINA4, PROZ, QSOX1, and SERPINA10;
APOB, APOC4, APOH, CDH1, PPBP, FABP1, AFP, FETUB, SERPINA4, and SERPINA10; and
APOB, APOC4, APOH, APOM, CAT, PPBP, FABP1, AFP, FETUB, ITIH1, SERPINA4, and SERPINA10.

12. The biomarker of any one of claims 1 to 3, wherein the subject at high risk of developing liver cancer is a liver cirrhosis patient, and the biomarker for monitoring or early diagnosis of liver cancer for the liver cirrhosis patient is a combination of the biomarkers selected from the group consisting of:
APOB, APOL1, CAT, CETP, C7, FABP1, AFP, FGA, FN1, IGHM, SERPINA5, ITIH2, SERPINA4, PON1, PON1, and SERPINA10;
BCO2, CAT, CETP, CFH, BCHE, AFP, FGA, SERPINA5, SELENOP, UCHL3, AZGP1, and SERPINA10;
AMBP, APOB, APOM, C1RL, CAT, CETP, CLU, PPBP, HSP90B1, F9, FGA, GSS, SERPINA5, SERPINA4, RBP4, HDLBP, AZGP1, and SERPINA10;
APOA4, APOB, C1RL, CETP, AFP, FGB, SERPIND1, SERPINA5, SERPINA4, LUM, QSOX1, SHBG, and SERPINA10;
APOA4, APOB, APOL1, CETP, C9, FBLN1, AFP, FGA, SERPINA5, ITIH2, and SERPINA10;
SERPINF2, APOB, CETP, FBLN1, AFP, FGA, SERPINA5, RBP4, HDLBP, and SERPINA10;
APOB, CETP, AFP, FGA, SERPINA5, SERPINA4, F2, and SERPINA10; and
APOA4, APOB, APOL1, CA2, CETP, CLU, C6, AFP, FGA, SERPINA5, ITIH2, SERPINA4, AZGP1, and SERPINA10.

13. The biomarker of any one of claims 1 to 3, wherein the biomarker is a combination of the biomarker selected from the group consisting of: APOB, CAT, CETP, C7, AFP, FETUB, FGA, GP5, SERPIND1, SERPINA5, ITIH2, SERPINA4, LGALS3BP, MPO, QSOX1, SHBG, TF, VTN, AZGP1 and SERPINA10; and ALB, APOB, CA1, CETP, C3, FCGBP, AFP, FETUB, FGA, JCHAIN, SERPINA5, ITIH2, SERPINA4, PRDX2, SELENOP, SHBG, THBS1, UCHL3, and SERPINA10.

14. The biomarker of any one of claims 1 to 3, wherein the subject at high risk of developing liver cancer is a hepatitis patient, and the at least one biomarker for monitoring or early diagnosis of liver cancer for the hepatitis patient is a combination of the biomarkers selected from the group consisting of:
IGFALS, APOB, APOC3, PPBP, FABP1, FCGBP, AFP, ITIH1, SERPINA4, PROS1, and SERPINA10; and
ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10.

15. The biomarker of any one of claims 1 to 3, wherein the subject at high risk of developing liver cancer is a liver cirrhosis patient, and the at least one biomarker for monitoring or early diagnosis of liver cancer for the liver cirrhosis patient is a combination of the biomarkers selected from the group consisting of:
BCO2, CAT, CETP, CFH, BCHE, AFP, FGA, SERPINA5, SELENOP, UCHL3, AZGP1, and SERPINA10; and
APOA4, APOB, C1RL, CETP, AFP, FGB, SERPIND1, SERPINA5, SERPINA4, LUM, QSOX1, SHBG, and SERPINA10.

16. The biomarker of any one of claims 1 to 15, wherein the blood sample is a plasma, serum, or whole blood.

17. The biomarker of any one of claims 1 to 16, wherein the biomarker is analyzed by an ELISA, an assay using Dipstick Rapid kit, an MRM assay, a Microarray assay, a Nucleic acid amplification assay or an Immunoassay.

18. The biomarker of claim 17, wherein the biomarker is analyzed by an MRM assay, wherein the peptides for each biomarker used for the MRM assay are the ones listed in Table 1-1. 1-2 and 1-3.

19. A method of monitoring or early diagnosis of liver cancer in a subject at high risk of developing liver cancer, comprising the steps of:
detecting or quantifying in a blood sample from the subject in need thereof at least one biomarker selected from the group consisting of ORM1 (Alpha-1-acid glycoprotein 1), SERPINA1 (Alpha-1-antitrypsin), SERPINF2 (Alpha-2-antiplasmin), A2M (Alpha-2-macroglobulin), ALB (Serum albumin), SERPINC1 (Antithrombin-III), APOA1 (Apolipoprotein A-I), APOA4 (Apolipoprotein A-IV), APOB (Apolipoprotein B-100), APOC3 (Apolipoprotein C-III), APOC4 (Apolipoprotein C-IV), APOF (Apolipoprotein F), APOH (Beta-2-glycoprotein 1), APOL1 (Apolipoprotein L1), APOM (Apolipoprotein M), BCO2 (Beta,beta-carotene 9',10'-oxygenase), BTD (Biotinidase), C1RL (Complement C1r subcomponent-like protein), CDH1 (Cadherin-1), CA1 (Carbonic anhydrase 1), CA2 (Carbonic anhydrase 2), CAT (Catalase), CPB2 (Carboxypeptidase B2), CETP (Cholesteryl ester transfer protein), CFH (Complement factor H), C3 (Complement C3), C8B (Complement component C8 beta chain), C9 (Complement component C9), PPBP (Platelet basic protein), HSP90B1 (Endoplasmin), ENTPD5 (Ectonucleoside triphosphate diphosphohydrolase 5), ESYT1 (Extended synaptotagmin-1), F7 (Coagulation factor VII), F9 (Coagulation factor IX), FABP1 (Fatty acid-binding protein, liver), FCGBP (IgGFc-binding protein), FETUB (Fetuin-B), FGA (Fibrinogen alpha chain), FGB (Fibrinogen beta chain), RACK1 (Receptor of activated protein C kinase 1), GP5 (Platelet glycoprotein V), GSS (Glutathione synthetase), SERPIND1 (Heparin cofactor 2), ICAM1 (Intercellular adhesion molecule 1), IGHM (Immunoglobulin heavy constant mu), IL1RAP (Interleukin-1 receptor accessory protein), ITIH2 (Inter-alpha-trypsin inhibitor heavy chain H2), ITIH3 (Inter-alpha-trypsin inhibitor heavy chain H3), KNG1 (Kininogen-1), LMNB1 (Lamin-B1), MPO (Myeloperoxidase), PON1 (Serum paraoxonase/arylesterase 1), PRDX2 (Peroxiredoxin-2), PROC (Vitamin K-dependent protein C), PROS1 (Vitamin K-dependent protein S), PROZ (Vitamin K-dependent protein Z), PSMD1 (26S proteasome non-ATPase regulatory subunit 1), PVR (Poliovirus receptor), RBP4 (Retinol-binding protein 4), S100A13 (Protein S100-A13), SAA4 (Serum amyloid A-4 protein), UBA2 (SUMO-activating enzyme subunit 2), SELENOP (Selenoprotein P), SPARC (SPARC), TF (Serotransferrin), THBS1 (Thrombospondin-1), UCHL3 (Ubiquitin carboxyl-terminal hydrolase isozyme L3), VCAM1 (Vascular cell adhesion protein 1), HDLBP (Vigilin), VIM (Vimentin), AZGP1 (Zinc-alpha-2-glycoprotein), and SERPINA10 (Protein Z-dependent protease inhibitor);
Comparing the result of the detection or the quantification to that from a control sample; and
Determining that the subject has an early stage of liver cancer when the result is changed in comparison to that of the control.

20. The method of claim 19, wherein the biomarker further comprises at least one biomarker selected from the group consisting of LRG1 (Leucine-rich alpha-2-glycoprotein), IGFALS (Insulin-like growth factor-binding protein complex acid labile subunit), AMBP (Protein AMBP [Cleaved into: Alpha-1-microglobulin), BCHE (Cholinesterase), CLU (Clusterin), CNDP1 (Beta-Ala-His dipeptidase), C6 (Complement component C6), C7 (Complement component C7), FBLN1 (Fibulin-1), FCGR3A (Low affinity immunoglobulin gamma Fc region receptor III-A), FCN3 (Ficolin-3), AFP (Alpha-fetoprotein), FN1 (Fibronectin), IGFBP3 (Insulin-like growth factor-binding protein 3), IGF2 (Insulin-like growth factor II), JCHAIN (Immunoglobulin J chain), SERPINA5 (Plasma serine protease inhibitor), ITIH1 (Inter-alpha-trypsin inhibitor heavy chain HI), SERPINA4 (Kallistatin), KLKB1 (Plasma kallikrein), LCAT (Phosphatidylcholine-sterol acyltransferase), LGALS3BP (Galectin-3-binding protein), LUM (Lumican), PLG (Plasminogen), QSOX1 (Sulfhydryl oxidase 1), SHBG (Sex hormone-binding globulin), F2 (Prothrombin), GC (Vitamin D-binding protein) and VTN (Vitronectin).

21. The method of claims 19 or 20, wherein the subject at high risk of developing liver cancer includes a hepatitis patient or a liver cirrhosis patient.

22. The method of any one of claims 19 to 21, wherein the at least one biomarker is selected from the group consisting of SERPINA1, ALB, APOB, APOC3, APOC4, APOL1, BTD, CDH1, CA1, CAT, CETP, C3, ESYT1, FCGBP, FETUB, FGA, GP5, GSS, SERPIND1, IL1RAP, ITIH2, LMNB1 MPO, PON1, PRDX2, PVR, SAA4, UBA2, SELENOP, TF, THBS1, UCHL3, HDLBP, AZGP1 and SERPINA10.

23. The method of claim 22, wherein the biomarker further comprises at least one biomarker selected from the group consisting of LRG1, C7, AFP, JCHAIN, SERPINA5, SERPINA4, LGALS3BP, LUM, QSOX1, SHBG, F2, and VTN.

24. The method of any one of claims 19 to 21, wherein the subject at high risk of developing liver cancer is a hepatitis patient, and the at least one biomarker for monitoring or early diagnosis of liver cancer for the hepatitis patient is selected from the group consisting of ORM1, A2M, ALB, SERPINC1, APOA1, APOA4, APOB, APOC3, APOC4, APOF, APOL1, APOM, APOH, BCO2, CDH1, CPB2, CA1, CA2, CAT, CETP, C8B, PPBP, F9, FABP1, ENTPD5, F7, FCGBP, FETUB, FGA, RACK1, GP5, SERPIND1, ICAM1, IGF2, IL1RAP, ITIH2, ITIH3, KNG1, PON1, PROC, PROS1, PROZ, PSMD1, RBP4, S100A13, SAA4, SELENOP, SPARC, THBS1, VCAM1, VIM and SERPINA10.

25. The method of claim 24, wherein the biomarker for monitoring or early diagnosis of liver cancer for the hepatitis patient further comprises at least one biomarker selected from the group consisting of IGFALS, BCHE, CLU, CNDP1, C7, FCGR3A, FCN3, AFP, IGFBP3, ITIH1, SERPINA4, KLKB1, LCAT, LGALS3BP, PLG, QSOX1, SHBG, F2, GC and VTN.

26. The method of any one of claims 19 to 21, wherein the subject at high risk of developing liver cancer is a liver cirrhosis patient, and the at least one biomarker for monitoring or early diagnosis of liver cancer for the liver cirrhosis patient is selected from the group consisting of SERPINF2, APOA4, APOB, APOL1, APOM, BCO2, C1RL, CA2, CAT, CETP, CFH, C9, PPBP, HSP90B1, F9, FABP1, FGA, FGB, GSS, SERPIND1, IGHM, ITIH2, MPO, PON1, QSOX1, RBP4, SELENOP, UCHL3, HDLBP, AZGP1 and SERPINA10.

27. The method of claim 26, wherein the biomarker for monitoring or early diagnosis of liver cancer for the liver cirrhosis patient further comprises at least one biomarker selected from the group consisting of AMBP, BCHE, CLU, C6, C7, FBLN1, AFP, FN1, SERPINA5, SERPINA4, LUM, SHBG, and F2.

28. The method of any one of claims 19 to 21, wherein the at least one biomarker is a combination of the biomarker selected from the group consisting of:
ALB, APOB, APOL1, BTD, CDH1, CAT, CETP, ESYT1, AFP, FGA, GP5, GSS, SERPINA5, ITIH2, ITIH2, ERPINA4, LUM, MPO, PON1, PVR, SAA4, and SERPINA10;
APOB, CAT, CETP, C7, AFP, FETUB, FGA, GP5, SERPIND1, SERPINA5, ITIH2, SERPINA4, LGALS3BP, MPO, QSOX1, SHBG, TF, VTN, AZGP1, and SERPINA10;
LRG1, APOC3, CETP, AFP, SERPINA5, TF, and SERPINA10;
APOC4, AFP, SERPINA5, LMNB1, MPO, PVR, and SERPINA10;
SERPINA1, APOB, CAT, CETP, AFP, FETUB, FGA, GSS, SERPINA5, ITIH2, SERPINA4, UBA2, SHBG, F2, THBS1, THBS1, and SERPINA10;
CETP, C7, AFP, IL1RAP, SERPINA5, LGALS3BP, MPO, HDLBP, and SERPINA10;
ALB, APOB, CA1, CETP, C3, FCGBP, AFP, FETUB, FGA, JCHAIN, SERPINA5, ITIH2, SERPINA4, PRDX2, SELENOP, SHBG, THBS1, UCHL3, and SERPINA10; and
ALB, APOB, CA1, CETP, AFP, FETUB, SERPINA5, ITIH2, SERPINA4, MPO, F2, THBS1, and SERPINA10.

29. The method of any one of claims 19 to 21, wherein the subject at high risk of developing liver cancer is a hepatitis patient, and the at least one biomarker for monitoring or early diagnosis of liver cancer for the hepatitis patient is a combination of biomarkers selected from the group consisting of:
IGFALS, APOA4, C7, PPBP, F9, FABP1, FCGBP, AFP, ITIH1, SERPINA4, and SERPINA10;
IGFALS, APOB, APOC3, PPBP, FABP1, FCGBP, AFP, ITIH1, SERPINA4, PROS1, and SERPINA10;
ALB, IGFALS, CETP, C7, PPBP, FABP1, AFP, IL1RAP, ITIH1, ITIH2, SERPINA4, S100A13, and SERPINA10;
ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10;
IGFALS, CA2, CETP, C7, PPBP, FABP1, FCGBP, FCN3, AFP, FGA, ITIH1, SERPINA4, and SERPINA10;
IGFALS, SERPINC1, APOA1, CAT, C7, PPBP, F9, FCGBP, AFP, FGA, ITIH2, SERPINA4, PROZ, QSOX1, and SERPINA10;
APOB, APOC4, APOH, CDH1, PPBP, FABP1, AFP, FETUB, SERPINA4, and SERPINA10; and
APOB, APOC4, APOH, APOM, CAT, PPBP, FABP1, AFP, FETUB, ITIH1, SERPINA4, and SERPINA10.

30. The method of any one of claims 19 to 21, wherein the subject at high risk of developing liver cancer is a liver cirrhosis patient, and the at least one biomarker for monitoring or early diagnosis of liver cancer for the liver cirrhosis patient is a combination of biomarkers selected from the group consisting of:
APOB, APOL1, CAT, CETP, C7, FABP1, AFP, FGA, FN1, IGHM, SERPINA5, ITIH2, SERPINA4, PON1, PON1, and SERPINA10;
BCO2, CAT, CETP, CFH, BCHE, AFP, FGA, SERPINA5, SELENOP, UCHL3, AZGP1, and SERPINA10;
AMBP, APOB, APOM, C1RL, CAT, CETP, CLU, PPBP, HSP90B1, F9, FGA, GSS, SERPINA5, SERPINA4, RBP4, HDLBP, AZGP1, and SERPINA10;
APOA4, APOB, C1RL, CETP, AFP, FGB, SERPIND1, SERPINA5, SERPINA4, LUM, QSOX1, SHBG, and SERPINA10;
APOA4, APOB, APOL1, CETP, C9, FBLN1, AFP, FGA, SERPINA5, ITIH2, and SERPINA10;
SERPINF2, APOB, CETP, FBLN1, AFP, FGA, SERPINA5, RBP4, HDLBP, and SERPINA10;
APOB, CETP, AFP, FGA, SERPINA5, SERPINA4, F2, and SERPINA10; and
APOA4, APOB, APOL1, CA2, CETP, CLU, C6, AFP, FGA, SERPINA5, ITIH2, SERPINA4, AZGP1, and SERPINA10.

31. The method of any one of claims 19 to 21, wherein the at least one biomarker is a combination of the biomarker selected from the group consisting of: APOB, CAT, CETP, C7, AFP, FETUB, FGA, FGA, GP5, SERPIND1, SERPINA5, ITIH2, SERPINA4, LGALS3BP, MPO, QSOX1, SHBG, TF, VTN, AZGP1 and SERPINA10; and ALB, APOB, CA1, CETP, C3, FCGBP, AFP, FETUB, FGA, JCHAIN, SERPINA5, ITIH2, SERPINA4, PRDX2, SELENOP, SHBG, THBS1, UCHL3, and SERPINA10.

32. The method of any one of claims 19 to 21, wherein the subject at high risk of developing liver cancer is a hepatitis patient, and the at least one biomarker for monitoring or early diagnosis of liver cancer for the hepatitis patient is a combination of biomarkers selected from the group consisting of:
IGFALS, APOB, APOC3, PPBP, FABP1, FCGBP, AFP, ITIH1, SERPINA4, PROS1, and SERPINA10; and ALB, APOB, BCO2, CDH1, CA1, BCHE, C7, PPBP, FABP1, FCGBP, AFP, FETUB, ITIH1, SERPINA4, KNG1, LGALS3BP, PSMD1, VCAM1, and SERPINA10.

33. The method of any one of claims 19 to 21, wherein the subject at high risk of developing liver cancer is a liver cirrhosis patient, and the at least one biomarker for monitoring or early diagnosis of liver cancer for the liver cirrhosis patient is a combination of biomarkers selected from the group consisting of:
BCO2, CAT, CETP, CFH, BCHE, AFP, FGA, SERPINA5, SELENOP, UCHL3, AZGP1, and SERPINA10; and APOA4, APOB, C1RL, CETP, AFP, FGB, SERPIND1, SERPIND1, SERPINA5, SERPINA4, LUM, QSOX1, SHBG, and SERPINA10.

34. The method of any one of claims 19 to 33, wherein the control sample is a sample from a hepatitis B patients or cirrhosis patients.

35. The method of any one of claims 19 to 33, wherein the step of detecting or quantifying the biomarker is performed by ELISA, Dip stick rapid analysis, Mass spectrometry, Microarray, Nucleic acid amplification, or immunoassay.

36. The method of claim 36, wherein the Mass spectrometry is an MRM (Multiple Reaction Monitoring), and the peptides for the MRM assay for each marker is the one listed in Table 1-1, 1-2 and 1-3.

37. A kit for early diagnosis or monitoring of the development of liver cancer of a subject at high risk of developing liver cancer comprising an agent for detecting the biomarker according to any one of claims 1 to 18.

38. A composition for early diagnosis or monitoring of the development of liver cancer of a subject at high risk of developing liver cancer comprising an agent for detecting the biomarker according to any one of claims 1 to 18.

39. A kit for early diagnosis or monitoring of the development of liver cancer of a subject at high risk of developing liver cancer comprising an agent for detecting the biomarker according to any one of claims 1 to 18 by the method according to any one of claims of 19 to 36.

40. A method of detecting *in vitro* the biomarker according to any one of claims 1 to 18.
